# EUROPEAN PATENT APPLICATION

(11) **EP 2 573 566 A1**
(43) Date of publication of application: **27.03.2013**
(21) Application number: 11182054.4
(22) Date of filing: 20.09.2011
(51) Int. Cl.: G01N 33/574

(54) **RBM3 in prostate cancer prognostics**

(71) Applicant: Atlas Antibodies AB, 106 91 Stockholm (SE)
(72) Inventor: Jirström, Karin, 216 18 Limhamn (SE)
(74) Representative: Wirén, Anders

(57) **Abstract**

There is provided a method of determining whether a mammalian subject having a prostate cancer belongs to a first or a second group, wherein the prognosis of subjects of the first group is better than the prognosis of subjects of the second group, comprising the steps of:
a) evaluating an amount of RBM3 in at least part of an earlier obtained sample comprising tumor cells from the prostate of said subject and determining a sample value corresponding to the evaluated amount;
b) comparing said sample value with a predetermined reference value;
and
if said sample value is higher than said reference value,
c1) concluding that the subject belongs to the first group; and
if said sample value is lower than or equal to said reference value,
c2) concluding that the subject belongs to the second group.

SEQUENCE LISTING

<110>  Atlas Antibodies AB

<120>  RBM3 IN PROSTATE CANCER PROGNOSTICS

<130>  11431ep

<160>  26

<170>  PatentIn version 3.5

<210>  1
<211>  134
<212>  PRT
<213>  Homo sapiens

<400>  1

Asp Glu Gln Ala Leu Glu Asp His Phe Ser Ser Phe Gly Pro Ile Ser
1               5                   10                  15

Glu Val Val Val Val Lys Asp Arg Glu Thr Gln Arg Ser Arg Gly Phe
            20                  25                  30

Gly Phe Ile Thr Phe Thr Asn Pro Glu His Ala Ser Val Ala Met Arg
        35                  40                  45

Ala Met Asn Gly Glu Ser Leu Asp Gly Arg Gln Ile Arg Val Asp His
    50                  55                  60

Ala Gly Lys Ser Ala Arg Gly Thr Arg Gly Gly Gly Phe Gly Ala His
65                  70                  75                  80

Gly Arg Gly Arg Ser Tyr Ser Arg Gly Gly Gly Asp Gln Gly Tyr Gly
                85                  90                  95

Ser Gly Arg Tyr Tyr Asp Ser Arg Pro Gly Gly Tyr Gly Tyr Gly Tyr
            100                 105                 110

Gly Arg Ser Arg Asp Tyr Asn Gly Arg Asn Gln Gly Gly Tyr Asp Arg
        115                 120                 125

Tyr Ser Gly Gly Asn Tyr
    130

<210>  2
<211>  157
<212>  PRT
<213>  Homo sapiens

<400>  2

Met Ser Ser Glu Glu Gly Lys Leu Phe Val Gly Gly Leu Asn Phe Asn
1               5                   10                  15

Thr Asp Glu Gln Ala Leu Glu Asp His Phe Ser Ser Phe Gly Pro Ile
            20                  25                  30

Ser Glu Val Val Val Val Lys Asp Arg Glu Thr Gln Arg Ser Arg Gly
        35                  40                  45

Phe Gly Phe Ile Thr Phe Thr Asn Pro Glu His Ala Ser Val Ala Met
    50                  55                  60

Arg Ala Met Asn Gly Glu Ser Leu Asp Gly Arg Gln Ile Arg Val Asp
65                  70                  75                  80

His Ala Gly Lys Ser Ala Arg Gly Thr Arg Gly Gly Gly Phe Gly Ala
                85                  90                  95

His Gly Arg Gly Arg Ser Tyr Ser Arg Gly Gly Gly Asp Gln Gly Tyr
            100                 105                 110

Gly Ser Gly Arg Tyr Tyr Asp Ser Arg Pro Gly Gly Tyr Gly Tyr Gly
        115                 120                 125

Tyr Gly Arg Ser Arg Asp Tyr Asn Gly Arg Asn Gln Gly Gly Tyr Asp
    130                 135                 140

Arg Tyr Ser Gly Gly Asn Tyr Arg Asp Asn Tyr Asp Asn
145                 150                 155

<210>  3
<211>  1130
<212>  DNA
<213>  Homo sapiens

<400>  3
atgcgcaatg tggcccccta atggtggctg cgctgagcca gctcctcaga ttaccacctt      60

attggccgcc tttctcagct tttctgtagt tacccatatt ttgttcctct ttcttgtcta     120

ttttctgtgc tttttctctg ctttccgtct cgctattttc tcacatctcc attttctttc     180

tccttcctgc caccattctt catgttcttc ccacaggact tgaactgcca tgtcctctga     240

agaaggaaag ctcttcgtgg gagggctcaa ctttaacacc gacgagcagg cactggaaga     300

ccacttcagc agtttcggac ctatctctga ggtggtcgtt gtcaaggacc gggagactca     360

gcggtccagg ggttttggtt tcatcacctt caccaaccca gagcatgctt cagttgccat 420

gagagccatg aacggagagt ctctggatgg tcgtcagatc cgtgtggatc atgcaggcaa 480

gtctgctcgg ggaaccagag gaggtggctt tggggcccat gggcgtggtc gcagctactc 540

tagaggtggt ggggaccagg gctatgggag tggcaggtat tatgacagtc gacctggagg 600

gtatggatat ggatatggac gttccagaga ctataatggc agaaaccagg gtggttatga 660

ccgctactca ggaggaaatt acagagacaa ttatgacaac tgaaatgaga catgcacata 720

atatagatac acaaggaata atttctgatc caggatcgtc cttccaaatg gctgtattta 780

taaaggtttt tggagctgca ccgaagcatc ttattttata gtatatcaac cttttgtttt 840

taaattgacc tgccaaggta gctgaagacc ttttagacag ttccatcttt ttttttaaat 900

tttttctgcc tatttaaaga caaattatgg gacgtttgta gaacctgagt atttttcttt 960

ttaccagttt tttagtttga gctcttaggt ttattggagc tagcaataat tggttctggc 1020

aagtttggcc agactgactt caaaaaatta atgtgtatcc agggacattt taaaaacctg 1080

tacacagtgt ttattgtggt taggaagcaa tttcccaatg tacctataag 1130

<210> 4
<211> 29
<212> PRT
<213> Homo sapiens

<400> 4

Arg Gly Phe Gly Phe Ile Thr Phe Thr Asn Pro Glu His Ala Ser Val
1               5                   10                  15

Ala Met Arg Ala Met Asn Gly Glu Ser Leu Asp Gly Arg
            20                  25

<210> 5
<211> 24
<212> PRT
<213> Homo sapiens

<400> 5

Arg Ser Tyr Ser Arg Gly Gly Gly Asp Gln Gly Tyr Gly Ser Gly Arg
1               5                   10                  15

Tyr Tyr Asp Ser Arg Pro Gly Gly
            20

<210> 6
<211> 5
<212> PRT

<213>  Homo sapiens

<400>  6

Thr Phe Thr Asn Pro
1               5


<210>  7
<211>  10
<212>  PRT
<213>  Homo sapiens

<400>  7

Gly Thr Arg Gly Gly Gly Phe Gly Ala His
1               5                   10


<210>  8
<211>  10
<212>  PRT
<213>  Homo sapiens

<400>  8

Gly Phe Gly Ala His Gly Arg Gly Arg Ser
1               5                   10


<210>  9
<211>  5
<212>  PRT
<213>  Homo sapiens

<400>  9

Tyr Asp Arg Tyr Ser
1               5


<210>  10
<211>  9
<212>  PRT
<213>  Homo sapiens

<400>  10

Asp Glu Gln Ala Leu Glu Asp His Phe
1               5


<210>  11
<211>  7
<212>  PRT
<213>  Homo sapiens

<400>  11

Thr Asn Pro Glu His Ala Ser

1               5


<210>  12
<211>  10
<212>  PRT
<213>  Homo sapiens

<400>  12

Glu His Ala Ser Val Ala Met Arg Ala Met
1               5                   10


<210>  13
<211>  10
<212>  PRT
<213>  Homo sapiens

<400>  13

Arg Gly Gly Gly Phe Gly Ala His Gly Arg
1               5                   10


<210>  14
<211>  8
<212>  PRT
<213>  Homo sapiens

<400>  14

Gly Phe Gly Ala His Gly Arg Gly
1               5


<210>  15
<211>  9
<212>  PRT
<213>  Homo sapiens

<400>  15

Asp Tyr Asn Gly Arg Asn Gln Gly Gly
1               5


<210>  16
<211>  10
<212>  PRT
<213>  Homo sapiens

<400>  16

His Gly Arg Gly Arg Ser Tyr Ser Arg Gly
1               5                   10


<210>  17
<211>  7

<212>  PRT
<213>  Homo sapiens

<400>  17

Gly Ser Gly Arg Tyr Tyr Asp
1               5


<210>  18
<211>  6
<212>  PRT
<213>  Homo sapiens

<400>  18

Ser Gly Arg Tyr Tyr Asp
1               5


<210>  19
<211>  3
<212>  PRT
<213>  Homo sapiens

<400>  19

Asp Glu Gln
1


<210>  20
<211>  15
<212>  PRT
<213>  Homo sapiens

<400>  20

Thr Gln Arg Ser Arg Gly Phe Gly Phe Ile Thr Phe Thr Asn Pro
1               5                   10                  15


<210>  21
<211>  20
<212>  PRT
<213>  Homo sapiens

<400>  21

Thr Gln Arg Ser Arg Gly Phe Gly Phe Ile Thr Phe Thr Asn Pro Glu
1               5                   10                  15


His Ala Ser Val
            20


<210>  22
<211>  3
<212>  PRT

<213>  Homo sapiens

<400>  22

Phe Thr Asn
1



<210>  23
<211>  21
<212>  PRT
<213>  Homo sapiens

<400>  23

Cys Thr Gln Arg Ser Arg Gly Phe Gly Phe Ile Thr Phe Thr Asn Pro
1               5                   10                  15


Glu His Ala Ser Val
            20


<210>  24
<211>  15
<212>  PRT
<213>  Homo sapiens

<400>  24

Gly Phe Gly Phe Ile Thr Phe Thr Asn Pro Glu His Ala Ser Val
1               5                   10                  15


<210>  25
<211>  15
<212>  PRT
<213>  Homo sapiens

<400>  25

Thr Phe Thr Asn Pro Glu His Ala Ser Val Ala Met Arg Ala Met
1               5                   10                  15


<210>  26
<211>  10
<212>  PRT
<213>  Homo sapiens

<400>  26

Thr Phe Thr Asn Pro Glu His Ala Ser Val
1               5                   10

## Description

### Field of the present disclosure

The present disclosure relates to the field of prognosis and treatment of prostate cancer.

### Background

### Cancer

Cancer is one of the most common diseases, and a major cause of death in the western world. In general, incidence rates increase with age for most forms of cancer. As human populations continue to live longer, due to an increase of the general health status, cancer may affect an increasing number of individuals. The cause of most common cancer types is still largely unknown, although there is an increasing body of knowledge providing a link between environmental factors (dietary, tobacco smoke, UV radiation etc) as well as genetic factors (germ line mutations in "cancer genes" such as p53, APC, BRCA1, XP etc) and the risk for development of cancer.

No definition of cancer is entirely satisfactory from a cell biological point of view, despite the fact that cancer is essentially a cellular disease and defined as a transformed cell population with net cell growth and anti-social behavior. Malignant transformation represents the transition to a malignant phenotype based on irreversible genetic alterations. Although this has not been formally proven, malignant transformation is believed to take place in one cell, from which a subsequently developed tumor originates (the "clonality of cancer" dogma). Carcinogenesis is the process by which cancer is generated and is generally accepted to include multiple events that ultimately lead to growth of a malignant tumor. This multi-step process includes several rate-limiting steps, such as addition of mutations and possibly also epigenetic events, leading to formation of cancer following stages of precancerous proliferation. The stepwise changes involve accumulation of errors (mutations) in vital regulatory pathways that determine cell division, asocial behavior and cell death. Each of these changes may provide a selective Darwinian growth advantage compared to surrounding cells, resulting in a net growth of the tumor cell population. A malignant tumor does not only necessarily consist of the transformed tumor cells themselves but also surrounding normal cells which act as a supportive stroma. This recruited cancer stroma consists of connective tissue, blood vessels and various other normal cells, e.g., inflammatory cells, which act in concert to supply the transformed tumor cells with signals necessary for continued tumor growth.

The most common forms of cancer arise in somatic cells and are predominantly of epithelial origin, e.g., prostate, breast, colon, urothelium and skin, followed by cancers originating from the hematopoetic lineage, e.g., leukemia and lymphoma, neuroectoderm, e.g., malignant gliomas, and soft tissue tumors, e.g., sarcomas.

### Cancer diagnostics and prognostics

Microscopic evaluation of biopsy material from suspected tumors remains the golden standard for cancer diagnostics. To obtain a firm diagnosis, the tumor tissue is fixated in formalin, histo-processed and paraffin embedded. From the resulting paraffin block, tissue sections can be produced and stained using both histochemical, i.e., hematoxylin-eosin staining, and immunohistochemical (IHC) methods. Also, sections from frozen tissue material may be employed. (IHC allows for the detection of protein expression patterns in tissues and cells using specific antibodies.) The surgical specimen is then evaluated with pathology techniques, including gross and microscopic analysis. This analysis often forms the basis for assigning a specific diagnosis, i.e., classifying the tumor type and grading the degree of malignancy, of a tumor.

Malignant tumors can be categorized into several stages according to classification schemes specific for each cancer type. The most common classification system for solid tumors is the tumor-node-metastasis (TNM) staging system. The T stage describes the local extent of the primary tumor, i.e., how far the tumor has invaded and imposed growth into surrounding tissues, whereas the N stage and M stage describe how the tumor has developed metastases, with the N stage describing spread of tumor to lymph nodes and the M stage describing growth of tumor in other distant organs. Early stages include: T0-1, N0, M0, representing localized tumors with negative lymph nodes. More advanced stages include: T2-4, N0, M0, localized tumors with more widespread growth and T1-4, N1-3, M0, tumors that have metastasized to lymph nodes and T1-4, N1-3, M1, tumors with a metastasis detected in a distant organ. Staging of tumors is often based on several forms of examination, including surgical, radiological and histopathological analyses. In addition to staging, for most tumor types there is also a classification system to grade the level of malignancy. The grading systems rely on morphological assessment of a tumor tissue sample and are based on the microscopic features found in a given tumor. These grading systems may be based on the degree of differentiation, proliferation and atypical appearance of the tumor cells. Examples of generally employed grading systems include Gleason grading for prostatic carcinomas and the Nottingham Histological Grade (NHG) grading for breast carcinomas.

Accurate staging and grading is normally considered crucial when determining an adequate therapeutic strategy for a given cancer patient.

### Prostate cancer

World wide, prostate cancer is the second most common form of cancer in men, and in some countries, such as Sweden, it is the most common form. Around 0.7 million new cases of prostate cancer are identified globally each year, and this accounts for more than 11 % of all new cancer cases. However, incidence rates vary vastly across the world. One reason for this is that some countries have screening programs, and in these countries, the number of diagnosed cases has increased dramatically. However, PSA-screening, which is the predominant form of prostate cancer screening, has not been shown to have a clear impact on survival rates.

Also, genetics and diet are factors that influence incidence rates; prostate cancer is least common in South and East Asia, more common in Europe, and most common in black men from the US. The prognosis is relatively good, with a 5-year survival of about 90 %, and even higher in e.g. the USA, where prostate cancer screening may lead to an increase in prostate cancer incidence.

Prostate cancer is predominantly a disease of older men, in Sweden over 60% gets the diagnosis after the age of 70. This cancer is most likely slow growing, and the majority will ultimately die of other causes. However, a problem is that there are no markers to distinguish between the slow growing and more aggressive forms. This makes treatment selection difficult, and every year many people have the prostate surgically removed without a proper cause. Two possible complications associated with prostate removal, incontinence and impotence, make prostatectomy a difficult decision for the patient. Watchful waiting is an alternative strategy, but the risk is higher (see below).

### Prostate cancer diagnostics

Prostate specific antigen (PSA) and its isoforms are currently the only markers recommended for testing of prostate cancer. PSA is specifically expressed in prostate and found upregulated during malignant conditions, but it is also expressed in normal prostate tissue and found upregulated during benign conditions such as hyperplasia and prostatitis. In addition, the normal level of PSA may vary between people, so in order to get a conclusive diagnosis, analysis of tissue from biopsy material is needed. Currently, only 25-30% of all PSA-high cases are confirmed as prostate cancer, thus there is a high false positive rate for prostate cancer detection. A way to improve diagnostic accuracy may be to analyze the free-to-total PSA ratio (f/t PSA). The free PSA molecules are small and readily leak into the blood even at minor disturbances, whereas the presence of PSA bound to a larger molecule, such as a carrier protein, might indicate a more severe prostate tissue disorder. Thus, a low f/t PSA value might be an indicator of a higher risk for prostate cancer than a high PSA-level alone.

Even though PSA is an unreliable marker for prostate cancer, screening programs have nevertheless started in some countries, e.g. the USA. However, the question of screening is controversial, and no clear survival benefit of PSA screening can be stated.

Another diagnostic method is the digital rectal exam (DRE), where a physician palpates the prostate with a finger via the rectum. A problem with this diagnostic method is that when the prostate cancer is large enough to be palpable it is often not curable.

In cases of suspected prostate cancer, such as elevated PSA-levels, a biopsy is generally taken from the prostate, e.g. via the rectum. If using a biopsy gun, several samples may be obtained in seconds. A number of 8-12 biopsy samples may for example be taken. If the biopsy samples are negative for tumor cells, but the PSA-level is still elevated, a second round of biopsies are often performed. If the second round biopsies are also negative, patients are usually monitored regularly regarding PSA-levels, and more biopsies may be taken if the PSA-levels increase further.

In order to diagnose prostate cancer by examining a biopsy, it may be useful to look at biological markers in addition to morphology. High molecular weight cytokeratin and p63 protein expression are rare in prostate cancer and the absence of these proteins may thus indicate tumor tissue. The tumor marker AMACR may also be of interest. Since most cases of prostate cancer do not require treatment, it would, however, be of interest to differentiate between indolent and life threatening disease.

### Treatment of prostate cancer

In Sweden, about half of the patients with prostate cancer are treated with curative intent, either with surgery and/or radiation therapy. Approximately 30 % of those treated with curative intent in Sweden get recurrences. Patients with recurrences may be given hormonal treatment. Hormonal treatment may also be given instead of surgery or radiation therapy as first line treatment. This is normally considered to be an option in cases of metastatic disease, slow growing disease, or if the patient has other life-threatening illnesses. Prostate cancer is, at least initially, dependent on testosterone for growth, and the hormonal treatment may be surgical removal of the testicles or chemical prevention of testosterone from influencing the tumor.

Low-risk prostate cancer patients (see below) may be subject to the watchful waiting strategy, where the patient is closely monitored for disease progression, and is only treated when symptoms appear. The treatment is then often hormonal therapy, see above. The watchful waiting strategy might especially be an option if the life expectancy is less than 10 years, the tumor is slow growing, or the patient has other severe illnesses. If life expectancy is more than 10 years, curative surgery might be an option in the form of radical prostatectomy, where the prostate is removed. Neoadjuvant hormonal therapy may be given before the surgical procedure. A common side effect of radical prostatectomy is impotence. Radiation treatment may also be an option, either in the form of external beam radiation therapy, or in the form of brachytherapy. For low-risk patients, the brachytherapy may be performed by implanting small radioactive "seeds" into the prostate, where they will irradiate the prostate from within. These two therapeutic modalities may also be combined, and neoadjuvant or adjuvant hormonal therapy may also be given in relation with radiation therapy. The risk of impotence is generally lower after radiation therapy than after a surgical procedure.

Intermediate- and high risk patients may be treated by surgery or radiation therapy, but with higher doses than the low-risk patients. Radiation therapy may be given as neoadjuvant and/or adjuvant therapy to radical prostatectomy, and hormonal treatment may be given as neoadjuvant and/or adjuvant therapy to radiation therapy. Other therapeutic options include cryotherapy, whereby the prostate is destroyed by freezing, and high-intensity focused ultrasound, whereby either the tumor or the entire prostate is destroyed by heating.

Patients with metastatic disease may be treated with chemotherapy, and palliative radiation therapy may be given to patients with bone metastases.

### Prognostics and treatment predictive factors

Today, the 5-year survival is approximately 87 % in Sweden. Prostate cancer may, in many cases, be a slow growing disease, where many patients are diagnosed late in life and do not die from their disease.

Prognostic information may be obtained by analyzing biopsy material for a Gleason score, and also by measuring the PSA-level in serum.

The Gleason Grading System is used on biopsy material, in the form of tissue slides, for prognostic information. The Gleason scale is a grading from 1 to 5, where 1 is the lowest grade, representing well differentiated cells that closely resembles normal prostate, and grade 5 represents poorly differentiated cells and tissue without recognizable glands. A Gleason score is then obtained by adding the grade for the two most common tumor patterns, and this score, together with the PSA level (see below), is normally what constitutes the prognostic information.

The PSA-level, an aid in diagnosing prostate cancer, may also provide prognostic information. High levels of PSA indicate a more aggressive cancer.

There are four different stages of prostate cancer. In stage I the cancer is confined to the prostate, has low grade (G), and is not visible by imaging (T1 a, N0, M0, G1), in stage II the cancer is more advanced, but is still confined within the prostate (T1 a-c,or T1-2, N0, M0, any G), in stage III disease the tumor has spread outside the prostate to nearby tissue (T3, N0, M0, any G), and in stage IV the tumor has metastasized to lymph nodes or other parts of the body such as bladder, rectum, bone, liver or lungs (any T, any N, or M1, any G).

The stage and grade of a prostate cancer is normally established by analysis of tissue from prostate biopsies.

Prostate cancer patients may be divided into three risk categories: Low, intermediate, and high risk. The low risk group are identified as those with a PSA concentration of < 10 ng/ml and a Gleason score (GS) of ≤ 6. The intermediate risk group is the patients having PSA levels of 10-20 ng/ml or a GS of 7, and the high risk group is the patients having PSA levels of > 20 ng/ml or a GS of 8-10. In the low and intermediate risk groups survival is high even without treatment with a 10-year survival of 80-90 %.

Nomograms can be used to calculate the estimated risk of the individual patient. The predictions are based on the experience of large groups of patients suffering from cancers at various stages. Various disease factors can be included in the nomograms, such as for example stage, PSA-level, biopsy pathology, use of hormone therapy, and radiation dosage.

### Summary of the present disclosure

There is an object of some aspects of the present disclosure to provide for the establishment of a prognosis, in particular a stage-independent prognosis, for prostate cancer.

Also, there is an object of some aspects of the present disclosure to provide for the personalization of prostate cancer treatment.

The following is a non-limiting and itemized listing of embodiments of the present disclosure, presented for the purpose of providing various features and combinations provided by the invention in certain of its aspects.
1. Method for determining whether a mammalian subject having a prostate cancer belongs to a first or a second group, wherein the prognosis of subjects of the first group is better than the prognosis of subjects of the second group, comprising the steps of:
   a) evaluating an amount of RBM3 in at least part of an earlier obtained sample comprising tumor cells from the prostate of said subject and determining a sample value corresponding to the evaluated amount;
   b) comparing said sample value with a predetermined reference value;
      and
      if said sample value is higher than said reference value,
   c1) concluding that the subject belongs to the first group; and if said sample value is lower than or equal to said reference value,
   c2) concluding that the subject belongs to the second group.
2. Method according to claim 1, wherein the prognosis is a stage-independent prognosis.
3. Method for determining whether a mammalian subject is not in need of a prostate cancer treatment regimen, comprising the steps of:
   a) evaluating an amount of RBM3 in at least part of an earlier obtained sample comprising tumor cells from the prostate of said subject and determining a sample value corresponding to the evaluated amount;
   b) comparing the sample value with a predetermined reference value;
      and
      if the sample value is higher than the reference value,
   c) concluding that the subject is not in need of said prostate cancer treatment regimen.
4. Non-treatment strategy method for a mammalian subject having a prostate cancer, comprising the steps of:
   a) evaluating an amount of RBM3 in at least part of an earlier obtained sample comprising tumor cells from the prostate of said subject and determining a sample value corresponding to the evaluated amount;
   b) comparing the sample value with a predetermined reference value;
      and
      if the sample value is higher than the reference value,
   c) refraining from treating the subject with a prostate cancer treatment regimen.
5. Method of treatment of a mammalian subject having a prostate cancer, comprising the steps of:
   a) evaluating an amount of RBM3 in at least part of an earlier obtained sample comprising tumor cells from the prostate of said subject and determining a sample value corresponding to the evaluated amount;
   b) comparing the sample value with a predetermined reference value;
      and
      if the sample value is lower than or equal to the reference value,
   c) treating the subject with a prostate cancer treatment regimen.
6. Method according to any one of the preceding items, wherein the first and the second group consists of subjects having prostate cancer of a stage which is the same as the stage of the prostate cancer of the subject of the method.
7. Method according to any one of the preceding items, wherein the subject has a PSA concentration of at least 10 ng/ml, such as at least 20 ng/ml, such as at least 30 ng/ml..
8. Method according to any one of the preceding items, wherein the subject has a Gleason sum of 5-8.
9. Method according to any one of the preceding items, wherein the TNM stage of the prostate cancer is T2-4, N0, M0.
10. Method according to any one of the preceding items, wherein said sample is obtained from a biopsy or surgically removed tissue.
11. Method according to any one of the preceding items, wherein the evaluation of step a) is limited to the nuclei of tumor cells of said sample.
12. Method according to any one of items 2-11, wherein said prostate cancer treatment regimen is selected from the group consisting of prostatectomy, cryosurgery, high-intensity focused ultrasound treatment and radiation therapy.
13. Method according to any one of items 2-11, wherein said prostate cancer treatment regimen is an adjuvant treatment.
14. Method according to item 13, wherein said adjuvant treatment is selected from the group consisting of chemotherapy, hormone therapy and adjuvant radiation therapy.
15. Method of determining whether a mammalian subject belongs to a first or a second group, wherein subjects of the first group have a higher risk of having a pre-stage of prostate cancer, such as prostate intraepithelial neoplasia (PIN), than subjects of the second group, comprising the steps of:
   a) evaluating an amount of RBM3 in at least part of an earlier obtained sample comprising biological material from the prostate of said subject and determining a sample value corresponding to the evaluated amount;
   b) comparing said sample value with a predetermined reference value;
      and
      if said sample value is higher than said reference value,
   c1) concluding that the subject belongs to the first group; and if said sample value is lower than or equal to said reference value,
   c2) concluding that the subject belongs to the second group.
16. Method according to item 15, wherein said sample comprises prostatic fluid or prostate tissue from the subject.
17. Method according to item 15 or 16, wherein the evaluation of step a) is limited to the nuclei of cells of said sample.
18. Method according to any one of items 15-17, wherein the subject has a PSA concentration of at least 10 ng/ml, such as at least 20 ng/ml.
19. Method according to any one of the preceding items, wherein said reference value is a value corresponding to a predetermined amount of RBM3 in a reference sample.
20. Method according to any one of the preceding items, wherein the sample value of step a) is determined as being either 1, corresponding to detectable RBM3 in the sample, or 0, corresponding to no detectable RBM3 in the sample.
21. Method according to any one of the preceding items, wherein the reference value of step b) corresponds to a reference sample having no detectable RBM3.
22. Method according to any one of the preceding items, wherein the reference value of step b) is 0.
23. Method according to any one of the preceding items, wherein said reference value is a nuclear fraction, a nuclear intensity or a function thereof.
24. Method according to item 23, wherein said reference value is a nuclear fraction of 0-50 %.
25. Method according to item 23, wherein said reference value is an absent, weak or moderate nuclear intensity.
26. Method according to any one of the preceding items, wherein step a) comprises:
   al) applying to said sample a quantifiable affinity ligand capable of selective interaction with the RBM3 protein to be evaluated, said application being performed under conditions that enable binding of the affinity ligand to RBM3 protein present in the sample; and
   all) quantifying the affinity ligand bound to said sample to evaluate said amount.
27. Method according to any one of items 1-25, wherein step a) comprises:
   a1) applying to said sample a quantifiable affinity ligand capable of selective interaction with the RBM3 protein to be quantified, said application being performed under conditions that enable binding of the affinity ligand to RBM3 protein present in the sample;
   a2) removing non-bound affinity ligand; and
   a3) quantifying affinity ligand remaining in association with the sample to evaluate said amount.
28. Method according to item 26 or 27, wherein the quantifiable affinity ligand is selected from the group consisting of antibodies, fragments thereof and derivatives thereof.
29. Method according to item 28, wherein said quantifiable affinity ligand is obtainable by a process comprising a step of immunizing an animal with a peptide whose amino acid sequence consists of a sequence selected from SEQ ID NO:4 and 5.
30. Method according to item 28 or 29, wherein said quantifiable affinity ligand is obtainable by a process comprising a step of immunizing an animal with an RBM3 fragment which consists of 21 amino acid residues or less, such as 15 amino acid residues or less, and comprises a sequence selected from SEQ ID NO:6-19 and 22-26.
31. Method according to any one of items 28-30, wherein said quantifiable affinity ligand is obtainable by a process comprising a step of immunizing an animal with an RBM3 fragment which consists of 20 amino acid residues or less, such as 15 amino acid residues or less, such as 10 amino acid residues or less and comprises a sequence selected from SEQ ID NO:8, 16, 17, 22, 24, 25 and 26.
32. Method according to item 26 or 27, wherein said quantifiable affinity ligand is an oligonucleotide molecule.
33. Method according to item 26 or 27, wherein the quantifiable affinity ligand is a protein ligand derived from a scaffold selected from the group consisting of staphylococcal protein A and domains thereof, lipocalins, ankyrin repeat domains, cellulose binding domains, γ crystallines, green fluorescent protein, human cytotoxic T lymphocyte-associated antigen 4, protease inhibitors, PDZ domains, peptide aptamers, staphylococcal nuclease, tendamistats, fibronectin type III domain and zinc fingers.
34. Method according to any one of items 26-33, wherein said quantifiable affinity ligand is capable of selective interaction with a peptide whose amino acid sequence consists of a sequence SEQ ID NO: 1.
35. Method according to any one of items 26-34, wherein said quantifiable affinity ligand is capable of selective interaction with a peptide consisting of an amino acid sequence selected from SEQ ID NO: 4 and 5.
36. Method according to any one of items 26-35, wherein said quantifiable affinity ligand is capable of selective interaction with an RBM3 fragment which consists of 21 amino acid residues or less, such as 15 amino acid residues or less, and comprises an amino acid sequence selected from SEQ ID NO: 6-19 and 22-26.
37. Method according to any one of items 26-36, wherein said quantifiable affinity ligand is capable of selective interaction with an RBM3 fragment which consists of 20 amino acid residues or less, such as 15 amino acid residues or less, and comprises a sequence selected from SEQ ID NO: 8, 16, 17, 22, 24, 25 and 26.
38. Method according to any one of items 26-37, wherein said quantifiable affinity ligand comprises a label selected from the group consisting of fluorescent dyes and metals, chromophoric dyes, chemiluminescent compounds and bioluminescent proteins, enzymes, radioisotopes, particles and quantum dots.
39. Method according to any one of items 26-38, wherein said quantifiable affinity ligand is detected using a secondary affinity ligand capable of recognizing said quantifiable affinity ligand.
40. Method according to item 39, wherein said secondary affinity ligand capable of recognizing said quantifiable affinity ligand comprises a label selected from the group consisting of fluorescent dyes and metals, chromophoric dyes, chemiluminescent compounds and bioluminescent proteins, enzymes, radioisotopes, particles and quantum dots.
41. Use *ex vivo* of an RBM3 protein as a prognostic marker for prostate cancer.
42. Use according to item 41, wherein said protein is provided in a sample from a subject having a prostate cancer.
43. Use according to item 42, wherein said sample is a prostate cancer tissue sample, e.g. derived from a biopsy.
44. Use according to any one of items 41-43, wherein said marker is a marker of a relatively good prostate cancer prognosis.
45. Use *ex vivo* of an RBM3 protein as a diagnostic marker for a pre-stage of prostate cancer, such as PIN.
46. Use according to item 45, wherein the RBM3 protein is provided in a prostate tissue sample.
47. Use according to item 45 or 46, wherein said prostate tissue sample is obtained from a biopsy.
48. Use *ex vivo* of an RBM3 protein, or an antigenically active fragment thereof, for the selection or purification of a prostate cancer prognostic agent.
49. Use *ex vivo* of an RBM3 protein, or an antigenically active fragment thereof, for the selection or purification of a diagnostic agent for the diagnosis of a pre-stage of prostate cancer.
50. Use of an RBM3 protein, or an antigenically active fragment thereof, for the production of a prostate cancer prognostic agent.
51. Use of an RBM3 protein, or an antigenically active fragment thereof, for the production of a diagnostic agent for the diagnosis of a pre-stage of prostate cancer.
52. Use according to any one of items 48-51, wherein said agent is an affinity ligand capable of selective interaction with the RBM3 protein or the antigenically active fragment thereof.
53. Use according any one of items 41-52, wherein the amino acid sequence of the RBM3 protein comprises a sequence selected from:
   i) SEQ ID NO: 1; and
   ii) a sequence which is at least 85 % identical to SEQ ID NO: 1.
54. Use according any one of items 41-52, wherein the amino acid sequence of the RBM3 protein comprises or consists of a sequence selected from:
   i) SEQ ID NO: 2; and
   ii) a sequence which is at least 85 % identical to SEQ ID NO:2.
55. Use of an antigenically active fragment according to any one of items 48-52, wherein the fragment consists of 50 amino acid residues or less and comprises or consists of an amino acid sequence selected from SEQ ID NO: 4-19 and 22-26.
56. Use according to item 55, wherein the fragment consists of 29 amino acid residues or less.
57. Use according to item 56, wherein the fragment consists of 21 amino acid residues or less and comprises an amino acid sequence selected from SEQ ID NO: 6-19 and 22-26.
58. Use according to item 57, wherein the fragment consists of 20 amino acid residues or less and comprises an amino acid sequence selected from SEQ ID NO: 8, 16, 17, 22, 24, 25 and 26.
59. Use according to item 57 or 58, wherein the fragment consists of 15 amino acid residues or less, such as 10 amino acid residues or less.
60. Use *ex vivo* of an affinity ligand capable of selective interaction with an RBM3 protein as a prostate cancer prognostic agent.
61. Use *ex vivo* of an affinity ligand capable of selective interaction with an RBM3 protein as a diagnostic agent for the diagnosis of a pre-stage of prostate cancer.
62. Use according to item 60 or 61, wherein the affinity ligand is obtainable by a process comprising a step of immunizing an animal with a peptide whose amino acid sequence consists of sequence SEQ ID NO: 4 or 5 or a RBM3 protein fragment which consists of 21 amino acid residues or less, such as 15 amino acid residues or less, such as 10 amino acid residues or less, and comprises an amino acid sequence selected from SEQ ID NO: 6-19 and 22-26.
63. Use according to item 62, wherein the affinity ligand is obtainable by a process comprising a step of immunizing an animal with a peptide whose amino acid sequence consists of SEQ ID NO:5 or a RBM3 protein fragment which consists of 20 amino acid residues or less, such as 15 amino acid residues or less, such as 10 amino acid residues or less and comprises a sequence selected from SEQ ID NO: 8, 16, 17, 22, 24, 25 and 26.
64. Use according to item 60 or 61, wherein the affinity ligand is capable of selective interaction with a peptide whose amino acid sequence consists of SEQ ID NO:4 or 5 or an RBM3 fragment which consists of 21 amino acid residues or less, such as 15 amino acid residues or less, such as 10 amino acid residues or less and comprises an amino acid sequence selected from SEQ ID NO:6-19 and 22-26.
65. Use according to item 64, wherein the affinity ligand is capable of selective interaction with a peptide whose amino acid sequence consists of SEQ ID NO:5 or an RBM3 fragment which consists of 20 amino acid residues or less, such as 15 amino acid residues or less, such as 10 amino acid residues or less and comprises an amino acid sequence selected from SEQ ID NO: 8, 16, 17, 22, 24, 25 and 26.
66. RBM3 protein fragment, which consists of 17 amino acid residues or less and comprises a sequence selected from SEQ ID NO: 22, 24, 25 and 26.
67. RBM3 protein fragment according to item 66, which consists of 15 amino acid residues residues or less, such as 10 amino acid residues residues or less, such as 6 amino acid residues or less.
68. Affinity ligand capable of selective interaction with an RBM3 protein fragment consisting of 21 amino acid residues or less and comprising a sequence selected from SEQ ID NO: 22-26.
69. Affinity ligand according to item 68 capable of selective interaction with an RBM3 protein fragment consisting of the amino acid residues of SEQ ID NO: 24 or 25.
70. Affinity ligand according to item 68 or 69 capable of selective interaction with an RBM3 protein fragment consisting of 10 amino acid residues or less and comprising a sequence selected from SEQ ID NO: 22 and 26.
71. Affinity ligand according to item 70 capable of selective interaction with an RBM3 protein fragment consisting of 6 amino acid residues or less and comprising the sequence SEQ ID NO: 22.
72. Polyclonal or monoclonal antibody according to any one of items 68-71.

### Brief description of the figures

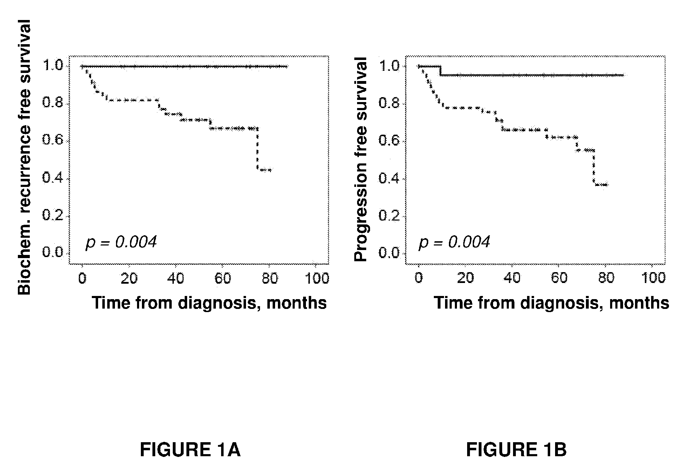

Figure 1A shows the impact of RBM3 level on biochemical recurrence (BCR) free survival of 88 patients diagnosed with prostate cancer. The solid line represents patients with tumors expressing high levels of RBM3 (nuclear score, NS > 2, n = 24) and the dotted line represents patients with tumors expressing low levels of RBM3 (NS ≤ 2, n = 64).
Figure 1 B shows the impact of RBM3 level on progression free survival of 88 patients diagnosed with prostate cancer. The solid line represents patients with tumors expressing high levels of RBM3 (nuclear score, NS > 2, n = 24) and the dotted line represents patients with tumors expressing low levels of RBM3 (NS ≤ 2, n = 64).

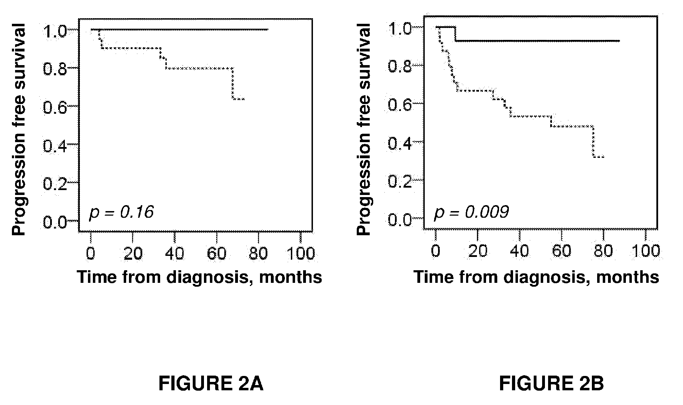

Figure 2A shows the impact of RBM3 level on progression free survival of 40 patients diagnosed with stage T1 prostate cancer. The solid line represents patients with tumors expressing high levels of RBM3 (nuclear score, NS > 2, n = 9) and the dotted line represents patients with tumors expressing low levels of RBM3 (NS ≤ 2, n = 31).
Figure 2B shows the impact of RBM3 level on progression free survival of 47 patients diagnosed with stage T2 prostate cancer. The solid line represents patients with tumors expressing high levels of RBM3 (nuclear score, NS > 2, n = 15) and the dotted line represents patients with tumors expressing low levels of RBM3 (NS ≤ 2, n = 32).

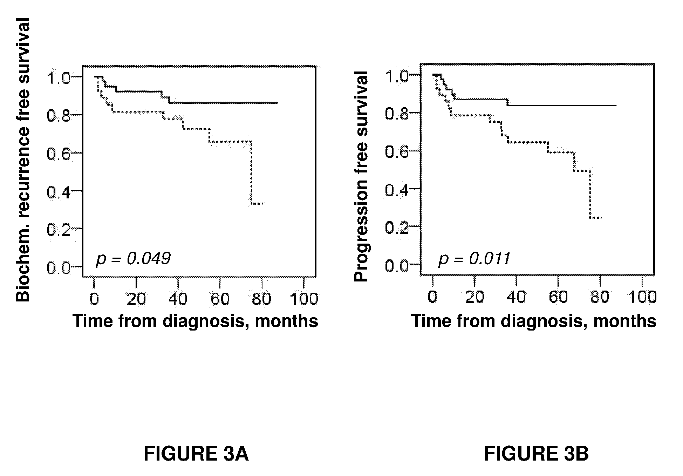

Figure 3A shows the impact of RBM3 level on biochemical recurrence (BCR) free survival of 88 patients diagnosed with prostate cancer. The solid line represents patients with tumors expressing RBM3 (nuclear score, NS > 0, n = 55) and the dotted line represents patients with tumors not expressing RBM3 (NS = 0, n = 33).
Figure 3B shows the impact of RBM3 level on progression free survival of 88 patients diagnosed with prostate cancer. The solid line represents patients with tumors expressing RBM3 (nuclear score, NS > 0, n = 55) and the dotted line represents patients with tumors not expressing RBM3 (NS = 0, n = 33).

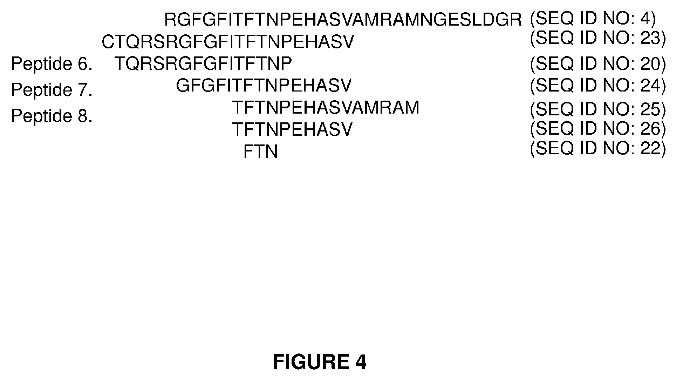

Figure 4 shows alignment of SEQ ID NO:4 as well as the peptides used for epitope mapping of the monoclonal antibodies.

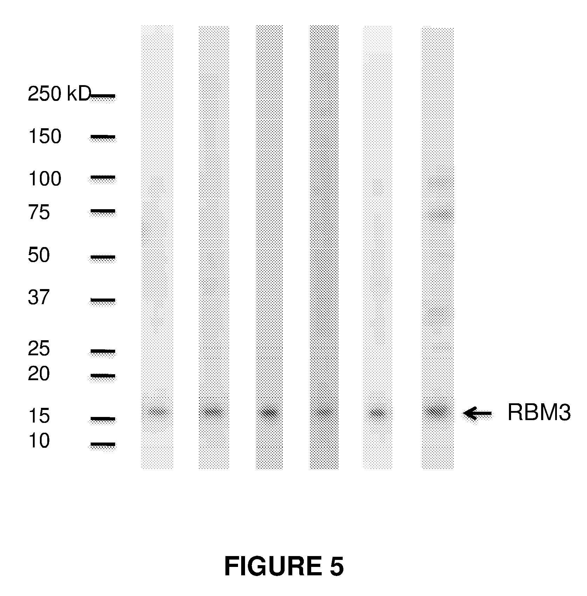

Figure 5 shows Western blot results for, from left to right, 7F5, 10F1, 12A10, 12C9, 14D9 and anti-RBM3. Thus, lanes 1 through 5 show the monoclonal antibodies while lane 6 shows the polyclonal anti-RBM3 antibody.

### Description

As a first aspect of the present disclosure, there is thus provided a method for determining whether a mammalian subject having a prostate cancer belongs to a first or a second group, wherein the prognosis of subjects of the first group is better than the prognosis of subjects of the second group, comprising the steps of:
a) evaluating an amount of RBM3 in at least part of an earlier obtained sample comprising tumor cells from the prostate of said subject and determining a sample value corresponding to the evaluated amount;
b) comparing said sample value with a predetermined reference value; and
   if said sample value is higher than said reference value,
c1) concluding that the subject belongs to the first group; and
   if said sample value is lower than or equal to said reference value,
c2) concluding that the subject belongs to the second group.

The present disclosure is based on the inventors' finding that the level of RBM3 is higher in samples from subjects having a more favorable prognosis than in samples from subjects having a less favorable prognosis. This difference is shown to be independent of previously recognized prognostic factors such as stage and Gleason score.

The incidence rate of prostate cancer is high and the outcome of the disease may eventually be death, especially if not treated in time, e.g. before metastasizing. However, some prostate cancer subjects can survive and maintain a good quality of life without treatment. Further, the side-effects of prostate cancer treatment are often severe, including incontinence, impotence and infertility. Also, the treatment in itself can be uncomfortable and/or painful.

Consequently, there is a clinical need for discriminating between non-aggressive (good prognosis) and aggressive (poor prognosis) prostate cancer forms. Accordingly, the method according to the first aspect may assist a diseased subject and his physician in the selection of an appropriate treatment (or non-treatment) strategy. For example, if a particularly aggressive form of the cancer is identified, an unpleasant treatment having severe side-effects may anyway be considered. Further, if less aggressive forms can be identified, over-treatment may be avoided.

Also, in case of a subject having a prostate cancer of an advanced stage, the additional prognostic information provided by the methods of the present disclosure may guide the physician (and the subject) deciding whether to apply an aggressive treatment in hope of prolonged survival or proceed with palliative treatment to relieve the subject from suffering during the remaining time. A high RBM3 level would here be in favor of the former alternative.

In addition, RBM3, as a marker for which a certain level of expression is correlated with a certain pattern of disease progression, has a great potential for example in a panel for making predictions or prognoses or for the selection of a treatment regimen.

The analysis of the level of RBM3 may be performed in connection with a biopsy or biopsy analysis. Thus, the level of RBM3 may be considered (e.g. together with the Gleason score and tumor stage) when assessing the risk when the presence of a prostate tumor has been established.

From the reasoning above, the benefits of the further aspects presented below will be evident to the person skilled in the art.

In the method of the first aspect, it is determined whether a prostate cancer subject belongs to a first or a second group, wherein subjects of the first group generally have a better prognosis than subjects of the second group. The division of prostate cancer subjects into the two groups is determined by comparing samples values from the subjects with a reference value. The reference value is thus the determinant for the size of the respective groups; the higher the reference value, the fewer the subjects in the first group and the lower the likelihood that a tested subject belongs to the first group. As the prognosis generally decreases as the sample value decreases, a relatively low reference value may in some instances be selected to identify subjects with a particularly poor prognosis. Guided by the present disclosure, the person skilled in the art may select relevant reference values without undue burden.

As the prognostic relevance of RBM3 in prostate cancer is found to be independent of a number of more or less established prognostic factors, the first and the second group may consist exclusively of subjects having prostate cancers of the same or similar grade (e.g. Gleason score), stage (e.g. TNM stage) and/or any other prognostic factor mentioned in Examples, section 1. Further, the groups may consist only of subjects having the same or similar age, race, sex, genetic characteristics and/or medical status or history.

It is also of particular clinical interest to find prognostic biomarkers that are independent of (and thereby complementary to) the prognostic information that may be obtained using conventional methods, such as grade and stage. For example, the clinical need for a biomarker that merely correlates with stage is limited as the determination of the stage is part of the routine examination protocol.

Thus, in preferred embodiments, the first and the second group consists of subjects having prostate cancer of a stage which is the same as the stage of the prostate cancer of the subject of the method.

This may also be expressed as that the prognosis is a stage-independent prognosis in the preferred embodiments.

Consequently, the subject and his doctor may use the method according to the first aspect to obtain additional information regarding the prognosis, which in turn may help them to make informed decisions regarding following actions.

The prognosis of the tested subject may also be determined relative to a reference prognosis. Accordingly, as a first configuration of the first aspect, there is provided a method for determining a prognosis for a mammalian subject having a prostate cancer, comprising the steps of:
a) evaluating an amount of RBM3 in at least part of an earlier obtained sample comprising tumor cells from the prostate of said subject and determining a sample value corresponding to the evaluated amount;
b) comparing said sample value with a reference value associated with a reference prognosis; and
   if said sample value is higher than said reference value,
c1) concluding that the prognosis for said subject is better than said reference prognosis; and/or
   if said sample value is lower than or equal to said reference value,
c2) concluding that the prognosis for said subject is worse than or equal to said reference prognosis.

However closely related and covered by the same concept, c1) and c2) provide two alternative conclusions.

Similarly and as a second configuration of the first aspect, there is provided a method for determining whether a prognosis for a mammalian subject having a prostate cancer is better than a reference prognosis, comprising the steps of:
a) evaluating an amount of RBM3 in at least part of an earlier obtained sample comprising tumor cells from the prostate of said subject and determining a sample value corresponding to the evaluated amount;
b) comparing the sample value obtained in step a) with a reference value associated with a reference prognosis; and,
   if said sample value is higher than said reference value,
c) concluding that the prognosis for said subject is better than said reference prognosis.

It follows that as a third configuration of the first aspect, there is provided a method for determining whether a prognosis for a mammalian subject having a prostate cancer is worse than or equal to a reference prognosis, comprising the steps of:
a) evaluating an amount of RBM3 in at least part of an earlier obtained sample comprising tumor cells from the prostate of said subject and determining a sample value corresponding to the evaluated amount;
b) comparing the sample value obtained in step a) with a reference value associated with a reference prognosis; and,
   if said sample value is lower than or equal to said reference value,
c) concluding that the prognosis for said subject is worse than or equal to said reference prognosis.

In the present disclosure, different RBM3 values (sample values) corresponding to various prognoses are presented. Typically, a low sample value is associated with a poorer prognosis than a high sample value. In the method of the third configuration of the first aspect, the sample value is compared to a reference value, and if the sample value is equal to or lower than the reference value, it is concluded that the prognosis for the subject is equal to, or worse than, a reference prognosis associated with the reference value.

Consequently, the method may be adapted to a reference value. In such case, starting from a sample value which under the circumstances is considered to be relevant, a reference value which is equal to the sample value may be selected. Subsequently, a reference prognosis associated with that reference value may be established. Guided by the present disclosure, the person skilled in the art understands how to establish a reference prognosis which corresponds to a given reference value. For example, the relation between sample values and outcome in a relevant group of cancer patients may be examined in line with what is described in Examples below. The procedure described therein may be adapted to a given reference value. Then, a prognosis corresponding to the given reference value may be selected as the reference prognosis.

Also, the method may be adapted to a given reference prognosis. In such case, starting from a reference prognosis which under the circumstances is considered to be relevant, for example for selecting an appropriate therapy, a corresponding reference value may be established. Guided by the present disclosure, the person skilled in the art understands how to establish a reference value which corresponds to a given reference prognosis. For example, the relation between sample values and outcome in a group of cancer patients may be examined as in Examples below, but the procedure described therein may be adapted to establish reference values corresponding to a given reference prognosis. For example, different reference values may be tested until one which correlates with the given reference prognosis is found.

The reasoning above applies *mutatis mutandis* to the other configurations of the first aspect.

Accordingly, the reference prognosis of the configurations of the first aspect may be based on a previously established prognosis, e.g., obtained by an examination of a relevant population of subjects. Such reference population may be selected to match the tested subject's age, sex, race, prostate cancer stage, prostate cancer type and/or medical status and history. Further, a prognosis may be adapted to a background risk in the general population, a statistical prognosis/risk or an assumption based on an examination of the subject. Such examination may also comprise the subject's age, sex, race, prostate cancer stage, prostate cancer type and/or medical status and history. Thus, a physician may for example adapt the reference prognosis to the subject's prostate cancer history, the type and/or stage of the tumor, the morphology of the tumor, the location of the tumor, the presence and spread of metastases and/or further cancer characteristics.

As an alternative configuration of the first aspect, there is provided a method for establishing a prognosis for a mammalian subject having a prostate cancer, comprising the steps of:
a) evaluating an amount of RBM3 in at least part of an earlier obtained sample comprising tumor cells from the prostate of said subject and determining a sample value corresponding to the evaluated amount; and
b) correlating the sample value of step a) to the prognosis for the subject. In the context of the present disclosure, "establishing a prognosis" refers to establishing a specific prognosis or a prognosis interval.

In an embodiment of the alternative configuration, the sample may be an earlier obtained sample.

The correlating of step b) refers to any way of associating outcome data to the obtained sample value so as to establish a prognosis for the subject.

The inventive concept of the present disclosure may also form the basis for a decision to refrain from a certain treatment regimen.

For example, the prognoses for subjects showing high RBM3 levels are generally better than those for subjects showing low RBM3 levels, as shown in the attached figures 1-3. Provided with the teachings of the present disclosure, a physician may consider the prognosis of an RBM3 high subject as being so favorable that certain treatment regimens are avoided and a less aggressive treatment regimen is selected instead. For example, the conclusion may be to refrain from an adjuvant treatment, such as hormonal treatment, if the subject is RBM3 high. As another example, the conclusion may be to refrain from surgery if the patient is RBM3 high.

In conclusion, the present disclosure may relieve subjects from over-treatment.

Thus, as a second aspect of the present disclosure, there is provided a method of determining whether a mammalian subject is not in need of a prostate cancer treatment regimen, comprising the steps of:
a) evaluating an amount of RBM3 in at least part of an earlier obtained sample comprising tumor cells from the prostate of said subject and determining a sample value corresponding to the evaluated amount;
b) comparing the sample value with a predetermined reference value;
   and
   if the sample value is higher than the reference value,
c) concluding that the subject is not in need of said prostate cancer treatment regimen.

In an embodiment, the conclusion may be that the subject is not in need of radical prostatectomy.

In another embodiment, the conclusion may be that the subject is not in need of *any* prostate cancer treatment at all. Instead, watchful waiting may be applied. In this embodiment, the prostate cancer has typically not metastasized. Thus, the prostate cancer of this embodiment may be of stage I-II or TNM stage T1-4, N0, M0.

These two embodiments apply to the configuration below *mutatis mutandis.*

Further, as a configuration of the second aspect, there is provided a non-treatment strategy method for a mammalian subject having prostate cancer, comprising the steps of:
a) evaluating an amount of RBM3 in at least part of an earlier obtained sample comprising tumor cells from the prostate of said subject and determining a sample value corresponding to the evaluated amount;
b) comparing the sample value with a predetermined reference value;
   and
   if the sample value is higher than the reference value,
c) refraining from treating the subject with a prostate cancer treatment regimen.

For example, step c) may be a refraining from the treatment regimen during at least one week from the completion of steps a) - b), such as at least one month from the completion of steps a) - b), such as at least three months from the completion of steps a) - b), such as at least six months from the completion of steps a) - b), such as at least one year from the completion of steps a) - b), such as at least two years from the completion of steps a) - b).

Alternatively, the refraining of step c) may be a refraining from treatment until the next time the method is performed or until a recurrence of a prostate cancer.

The inventive concept of the present disclosure may also form the basis for applying various treatment regimes.

Subjects having more aggressive prostate cancer are normally in need of treatment. As a third aspect of the present disclosure, there is thus provided a method of treatment of a mammalian subject having a prostate cancer, comprising the steps of:
a) evaluating an amount of RBM3 in at least part of an earlier obtained sample comprising tumor cells from the prostate of said subject and determining a sample value corresponding to the evaluated amount;
b) comparing the sample value with a predetermined reference value;
   and
   if the sample value is lower than or equal to the reference value,
c) treating the subject with a prostate cancer treatment regimen.

The method of treatment may be limited to the decision-making and treatment. Thus, as a configuration of the third aspect, there is provided a method of treatment of a mammalian subject having a prostate cancer, comprising the steps of:
α) comparing a sample value corresponding to a level of RBM3 in a sample comprising tumor cells from the prostate of said subject with a predetermined reference value; and
if the sample value is lower than or equal to the reference value, β) treating the subject with a prostate cancer treatment regimen.

Various ways of obtaining a sample value corresponding to a level of RBM3 in a sample are described in the present disclosure.

A subject may have a prostate cancer in such an advanced stage that an adjuvant therapy would normally be considered superfluous and unnecessary painful. However, in such case, a physician may anyway decide to apply the adjuvant therapy if the subject in question has an increased probability of prolonged survival due to a high RBM3 protein value.

Thus, as an alternative configuration of the third aspect, there is provided a method of treatment of a subject having a prostate cancer of an advanced stage, such as stage IV, comprising:
a) evaluating an amount of RBM3 in at least part of an earlier obtained sample comprising tumor cells from the prostate of said subject and determining a sample value corresponding to the evaluated amount;
b) comparing said sample value with a predetermined reference value; and
   if said sample value is higher than said reference value,
c) treating said subject with an adjuvant prostate cancer treatment regimen for prolonged survival.

Further, if said sample value is lower than or equal to said reference value, the subject may be treated with palliative treatment only.

The level RBM3 may also be considered when deciding the intensity of a prostate cancer treatment. As fourth aspect of the present disclosure, there is thus provided a method for determining the level of intensity of a treatment of a mammalian subject having a prostate cancer, comprising the steps of:
a) evaluating an amount of RBM3 in at least part of an earlier obtained sample comprising tumor cells from the prostate of said subject and determining a sample value corresponding to the evaluated amount;
b) comparing the sample value with a predetermined reference value; and
   if the sample value is lower than or equal to the reference value,
c1) concluding that said subject should be given a treatment of a first intensity; and
   if the sample value is higher than the reference value,
c2) concluding that said subject should be given a treatment of a second intensity;
wherein the first intensity is higher than the first intensity.

The level of intensity may for example be measured as the average daily or weekly dose of a therapeutic agent given to the subject. A treatment of the first intensity may thus be applied more frequently or in higher individual doses than a treatment of the second intensity. The treatment of the first intensity may also comprise application of a more aggressive therapy than the treatment of the second intensity. For example, the treatment of the first intensity may be a combination treatment while the treatment of the second intensity is a mono-therapy. Yet another possibility is that the treatment of the first intensity is applied for a longer period than the treatment of the second intensity.

In an embodiment, c1) may thus be concluding that said subject should undergo treatment during a first period and c2) may be concluding that said subject should undergo treatment during a second period, wherein the first period is longer than the second period.

In an alternative or complementary embodiment, c1) may thus be concluding that said subject should undergo first prostate cancer treatment regimen and c2) may be concluding that said subject should undergo second prostate cancer treatment regimen, wherein the first prostate cancer treatment regimen is more aggressive than the second prostate cancer treatment regimen. For a given prostate cancer subject, the person of skill in the art understands, under the circumstances, what to consider a more aggressive and a less aggressive treatment regimen, respectively.

The prostate cancer treatment or treatment regimen of the present disclosure may for example be selected from surgery, cryosurgery, high-intensity focused ultrasound radiation therapy, hormone therapy, chemotherapy, immunotherapy and combinations thereof.

The surgery may for example be retropubic prostatectomy, perineal prostatectomy or laparoscopic prostatectomy.

Cryosurgery (or cryoablation) is a treatment in which prostate cancer cells are freezed and destroyed. Cryosurgery is usually performed with a special instrument (normally comprising hollow needles/cryoprobes) that contains a cold fluid, such as liquid nitrogen or liquid carbon dioxide.

High-intensity focused ultrasound (ultrasonography) is a treatment that uses ultrasound (high-energy sound waves) to destroy cancer cells. To treat prostate cancer, an endorectal probe is used to make the sound waves; a procedure in which high-energy sound waves (ultrasound) are bounced off internal tissues or organs and make echoes. The echo patterns may be shown on a screen of an ultrasound machine, forming a picture of body tissues called a sonogram.

Radiation therapy is a cancer treatment that uses high-energy x-rays or other types of radiation to kill cancer cells or keep them from growing. There are mainly two types of radiation therapy that are considered in the methods of the present disclosure; external radiation therapy (e.g. 3-D conformal radiation therapy or intensity-modulated) and internal radiation therapy (brachytherapy). External radiation therapy uses a machine outside the body to send radiation towards the cancer. Internal radiation therapy uses a radioactive substance sealed in needles, seeds, wires, catheters or the like that are placed directly into or near the cancer. The way the radiation therapy is given depends on the type and stage of the cancer being treated.

Hormone therapy is a cancer treatment that removes hormones or blocks their action and thereby stops cancer cells from growing. Hormones are substances produced by glands in the body and circulated in the bloodstream. In prostate cancer, male sex hormones can cause prostate cancer to grow. The hormone therapy of the present disclosure may for example be:
application of a luteinizing hormone-releasing hormone agonist, such as leuprolide, goserelin or buserelin (such an agonist can prevent the testicles from producing testosterone);
application of an antiandrogen, such as flutamide or nilutamide (such an antiandrogen can block the action of androgens, i.e. hormones that promote male sex characteristics);
application of a drugs that can prevent the adrenal glands from making androgens, such as ketoconazole or aminoglutethimide;
orchiectomy, which is a surgical procedure to remove one or both testicles, the main source of male hormones, to decrease hormone production; and
application of an estrogen (hormones that promote female sex characteristics) can prevent the testicles from producing testosterone.

Chemotherapy is a cancer treatment that uses drugs to stop the growth of cancer cells, either by killing the cells or by stopping them from dividing. The chemotherapy of the present disclosure may for example be selected from Docetaxel, Mitozantrone, Epirubicin, Paclitaxel and Estramustine.

Biological therapy (sometimes referred to as biotherapy, immunotherapy or BRM therapy) is a treatment that uses the patient's immune system to fight cancer. Substances made by the body or made in a laboratory are mainly used to boost, direct, or restore the body's natural defenses against cancer, but can also be used to lessen certain side effects that may be caused by some cancer treatments. Agents used in biological therapy include (therapeutic) monoclonal antibodies known to the skilled person. These agents may have a direct antitumor effect.

The radiation therapy, hormone therapy, chemotherapy, immunotherapy, high-intensity focused ultrasound may be adjuvant or neoadjuvant.

In general, when deciding on a suitable treatment strategy for a patient having a prostate cancer, the physician responsible for the treatment may take several parameters into account, such as the result of an immunohistochemical evaluation, patient age, tumor type, stage and grade, general condition and medical history, such as prostate cancer history. To be guided in the decision, the physician may perform a RBM3 test, or order a RBM3 test performed, according to the above methods.

The steps of the above methods may be performed by a single person, such as a lab technician, nurse or physician. However, a physician may also perform step c) and optionally step b) himself, while assigning the performance of step a) and optionally step b) to someone else, typically a lab technician, nurse. Also, the physician responsible for the diagnosis and treatment may be one person, typically a urologist, while the physician performing the examination of the sample is another person, normally a pathologist. If the above methods involve more than one person, the persons may be situated at different locations and have different employers. For example, the physician may be employed by a hospital while the lab technician or pathologist is employed by a lab. Samples and information may thus be transferred from one person and location to another within the framework of the methods of the present disclosure.

In the context of the present disclosure, the "level of RBM3" refers to the level of RBM3 protein or the level of RBM3 mRNA. The levels of mRNA and protein expression of a certain gene are not always correlated. However, the inventors have noted that the levels of m RNA and protein expression are correlated for the RBM3 gene in various tissues. Further, unpublished observations support that the level of RBM3 mRNA and protein is correlated also in prostate tissue. In the Examples below, the RBM3 protein expression is measured and shown to be significantly correlated with prognosis. Thus, in preferred embodiments, "RBM3" refers to RBM3 protein.

The skilled person should recognize that the usefulness of the methods according to the above aspects is not limited to the quantification of any particular variant of the RBM3 protein or RBM3 mRNA present in the subject in question, as long as the protein is encoded by the relevant gene and presents the relevant pattern of expression.

As a non-limiting example, the RBM3 protein may comprise a sequence selected from:
i) SEQ ID NO:1; and
ii) a sequence which is at least 85 % identical to SEQ ID NO:1.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

As another non-limiting example, the RBM3 protein may comprise, or consists of, a sequence selected from:
i) SEQ ID NO:2; and
ii) a sequence which is at least 85 % identical to SEQ ID NO:2.

In some embodiments, sequence ii) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

The term "% identical", as used in the context of the present disclosure, is calculated as follows. The query sequence is aligned to the target sequence using the CLUSTAL W algorithm (Thompson, J.D., Higgins, D.G. and Gibson, T.J., Nucleic Acids Research, 22: 4673-4680 (1994)). The amino acid residues at each position are compared, and the percentage of positions in the query sequence that have identical correspondences in the target sequence is reported as % identical. Also, the target sequence determines the number of positions that are compared. Consequently, in the context of the present disclosure, a query sequence that is shorter than the target sequence can never be 100 % identical to the target sequence. For example, a query sequence of 85 amino acid residues may at the most be 85 % identical to a target sequence of 100 amino acid residues.

Regarding step a) of the methods of the present disclosure, an increase in the amount of RBM3 typically results in an increase in the sample value, and not the other way around. However, in some embodiments, the evaluated amount may correspond to any of a predetermined number of discrete sample values. In such embodiments, a first amount and a second, increased, amount may correspond to the same sample value. In any case, an increase in the amount of RBM3 protein will not result in a decrease in the sample value in the context of the present disclosure.

However inconvenient, but in an equivalent fashion, the evaluated amounts may be inversely related to sample values if the qualification between step b) and c) is inverted. For example, the qualification between step b) and c) is inverted if the phrase "if the sample value is higher than the reference value" is replaced with "if the sample value is lower than the reference value".

In the context of the present disclosure, "prognosis" refers to the prediction of the course or outcome of a disease and its treatment. For example, prognosis may also refer to a determination of chance of survival or recovery from a disease, as well as to a prediction of the expected survival time of a subject. A prognosis may specifically involve establishing the likelihood for survival of a subject during a period of time into the future, such as three years, five years, ten years or any other period of time. A prognosis may further be represented by a single value or a range of values.

Further, in the context of the methods of the present disclosure, "earlier obtained" refers to obtained before the method is performed. Consequently, if a sample earlier obtained from a subject is used in a method, the method does not involve obtaining the sample from the subject, i.e., the sample was previously obtained from the subject in a step separate from the method.

The methods and uses of the present disclosure, except the methods of treatment, may unless otherwise stated be carried out entirely *ex vivo.*

Further, in the context of the present disclosure, "a mammalian subject having a prostate cancer" refers to a mammalian subject having a primary prostate tumor or a mammalian subject which has had a primary prostate tumor removed, wherein the removal of the tumor refers to eradicating the tumor by any appropriate type of surgery or therapy. In the method and use aspects of the present disclosure, "a mammalian subject having a prostate cancer" also includes the cases wherein the mammalian subject is suspected of having a prostate cancer at the time of the use or the performance of the method and the prostate cancer diagnosis is established later.

Further, in the context of the present disclosure, the "predetermined reference value" refers to a predetermined value found to be relevant for making decisions or drawing conclusions regarding the prognosis or a suitable treatment strategy for the subject.

Also, in the context of the present disclosure, a reference value being "associated" with a reference prognosis refers to the reference value being assigned a corresponding reference prognosis, based on empirical data and/or clinically relevant assumptions. For example, the reference value may be the average RBM3 value in a relevant group of subjects and the reference prognosis may be an average/mean prognosis in the same group. Further, the reference value does not have to be assigned to a reference prognosis directly derived from prognosis data of a group of subjects exhibiting the reference value. The reference prognosis may for example correspond to the prognosis for subjects exhibiting the reference value or lower. That is, if the reference value is 1 on a scale from 0 to 2, the reference prognosis may be the prognosis of the subjects exhibiting the values 0 or 1. Consequently, the reference prognosis may also be adapted to the nature of the available data. As further discussed above, the reference prognosis may be further adapted to other parameters as well.

Step a) of the methods of the above aspects involve evaluating an amount of RBM3 present in at least part of the sample, and determining a sample value corresponding to the amount. The "at least part of the sample" refers to a relevant part or relevant parts of the sample for establishing the prognosis or drawing conclusions regarding suitable treatments. The person skilled in the art understands which part or parts that are relevant under the circumstances present when performing the method. For example, the skilled person may only consider the nuclei of tumor cells of the sample. Likewise, an automated analysis, such as an automated scoring system for stained tissue samples, may be adapted to take only the relevant part(s) into account.

Further, in step a) an amount is evaluated and a sample value corresponding to the amount is determined. Consequently, an exact measurement of the amount of RBM3 is not required for obtaining the sample value. For example, the amount of RBM3 may be evaluated by visual inspection of a prepared and stained tissue sample and the sample value may then be categorized as for example high or low based on the evaluated amount.

The person of skill in the art understands that the evaluation and determination of step a) involves some kind of processing or manipulation of the sample obtained from the subject. It is not possible to determine the sample value by mere visual inspection of the native sample. Various techniques, of which some are presented below, for such evaluation and determination are well known to the skilled person. The methods of the present disclosure are therefore not limited to any specific technique or techniques for the performance of step a).

In some cases, the additional prognostic information obtained according to the present disclosure may be particularly anticipated. An example of such a case is when a tumor has been found to be of intermediate risk using prior art methods because it may then be particularly difficult to decide whether or not to apply various treatments.

Subjects traditionally considered to have an intermediate risk prostate cancer normally have a PSA concentration of 10 ng/ml or more, a Gleason sum of 5-8 and/or a non-metastasizing T2-4 tumor.

Accordingly, the subject of the above methods may for example have a PSA concentration of at least 10 ng/ml, such as at least 20 ng/ml, such as at least 30 ng/ml. In an alternative or complementary embodiment, the subject may for example have Gleason sum of 5-8.

In another alternative or complementary embodiment, the TNM stage of the subject's prostate cancer may be T2-4, N0, M0.

When a subject's prostate is examined, one or more biopsies are often taken. The biopsy material is then used for a morphological evaluation and the determination of a Gleason score. These biopsies may also provide the samples of the above methods. Another source of sample material may be surgically removed tissue. Thus, the above methods may be performed using readily available sample material. Accordingly, in embodiments of the above methods, the sample may be obtained from a biopsy or surgically removed tissue.

RBM3 protein is found both in the nucleus and in the cytoplasm of cells. The inventors have however noted that nuclear expression of RBM3 protein is particularly relevant for determining prognoses or selecting treatments according to the present disclosure. Thus, the evaluation of step a) may be limited to the nuclei of tumor cells of the sample. Consequently, when a tissue sample is examined, only the nuclei of tumor cells may be taken into consideration. Such examination may for example be aided by immunohistochemical staining.

When performing the methods according to the above aspects, it may be convenient to use zero as the reference value, because in such case, it has only to be established in step a) whether RBM3 protein is present in the sample or not. The examples below show that zero (i.e. no detectable RBM3 protein) is a working cut-off value for establishing two subgroups of significantly different prognoses (see also figs 3A and 3B).

Thus, in embodiments of the methods of the above aspects, the sample value of step a) may be either 1, corresponding to detectable RBM3 protein in the sample, or 0, corresponding to no detectable RBM3 protein in the sample. Consequently, in such embodiments, the evaluation of the sample is digital: RBM3 protein is considered to be either present or not. In the context of the present disclosure, "no detectable RBM3 protein" refers to an amount of RBM3 protein that is so small that it is not, during normal operational circumstances, detectable by a person or an apparatus performing the step a). The "normal operational circumstances" refer to the laboratory methods and techniques a person skilled in the art would find appropriate for performing the methods of the present disclosure.

Accordingly, in embodiments of the methods of the present disclosure, the reference value of step b) may be 0. And it follows that, in further embodiment of the methods of the present disclosure, the reference value of step b) may correspond to a reference sample having no detectable RBM3 protein (see below).

A sample value of RBM3 being higher than the reference value, or a subject from which such sample value is obtained, is sometimes herein referred to as being "RBM3 high". Further, a sample value of RBM3 being lower than, or equal to, the reference value, or a subject from which such sample value is obtained, is sometimes herein referred to as being "RBM3 protein low".

In the context of the present disclosure, the terms "sample value" and "reference value" are to be interpreted broadly. The quantification of RBM3 to obtain these values may be done via automatic means, via a scoring system based on visual or microscopic inspection of samples, or via combinations thereof. However, it is also possible for a skilled person, such as a person skilled in the art of histopathology, to determine the sample and reference values by inspection, e.g., of tissue slides that have been prepared and stained for RBM3 protein expression.

Determining that the sample value is higher than the reference value may thus be determining, upon visual or microscopic inspection, that a sample tissue slide is more densely stained and/or exhibit a larger fraction of stained cells than a reference tissue slide. The sample value may also be compared to a reference value given by a literal reference, such as a reference value described in wording or by a reference picture. Consequently, the sample and/or reference values may in some cases be mental values that the skilled person envisages upon inspection and comparison.

One or more of the steps of the methods of the present disclosure may be implemented in an apparatus. For example, step a) and optionally step b) may be performed in an automatic analysis apparatus, and such an apparatus may be based on a platform adapted for immunohistochemical analysis. As an example, one or more tumor tissue sample(s) from the subject in question may be prepared for imunohistochemical analysis manually and then loaded into the automatic analysis apparatus, which gives the sample value of step a) and optionally also performs the comparison with the reference value of step b). The operator performing the analysis, the physician ordering the analysis or the apparatus itself may then draw the conclusion of step c). Consequently, software adapted for drawing the conclusion of step c) may be implemented on the apparatus.

A reference value, which is relevant for establishing a prognosis or making a treatment decision regarding prostate cancer subjects, for use as comparison with the sample value from the subject, may be provided in various ways. With the knowledge of the teachings of the present disclosure, the skilled artisan can, without undue burden, provide relevant reference values for performing the methods of the present disclosure.

The person performing the methods of the above aspects may, for example, adapt the reference value to desired information. For example, the reference value may be adapted to yield the most significant prognostic information, e.g., the largest separation between the outcome curves (see figures 1-3). Alternatively, the reference value may be selected such that a group of subjects having particularly good prognoses or particularly poor prognoses is singled out.

In embodiments of the methods of the above aspects, the reference value may correspond to the amount of RBM3 in a healthy tissue from the subject of the method. As another example, the reference value may be provided by the amount of RBM3 measured in a standard sample of normal tissue from another, comparable subject. As another example, the reference value may be provided by the amount of RBM3 measured in a reference sample comprising tumor cells, such as a reference sample of prostate tumor tissue. The amount of protein expression of the reference sample may preferably be previously established. Consequently, the reference value may be provided by the amount of RBM3 measured in a reference sample comprising cells expressing a predetermined amount of RBM3.

Further, the reference value may for example be provided by the amount of RBM3 protein expression measured in a reference sample comprising cell lines, such as cancer cell lines, expressing a predetermined, or controlled, amount of RBM3 protein. The person skilled in the art understands how to provide such cell lines, for example guided by the disclosure of Rhodes *et al.* (2006) *The biomedical scientist,* p 515-520.

Consequently, in embodiments of the methods of the present disclosure, the reference value may be a predetermined value corresponding to the amount of RBM3 in a reference sample.

However, as discussed further below, the amount of RBM3 protein in the reference sample does not have to directly correspond to the reference value. The reference sample may also provide an amount of RBM3 protein that helps a person performing the method to assess various reference values. For example, the reference sample(s) may help in creating a mental image of the reference value by providing a "positive" reference value and/or a "negative" reference value.

One alternative for the quantification of RBM3 protein expression in a sample, such as the sample earlier obtained from the subject or the reference sample, is the determination of the fraction of cells in the sample that exhibit RBM3 protein expression over a certain level. The fraction may for example be: a "cellular fraction", wherein the RBM3 protein expression of the whole cells is taken into account; a "cytoplasmic fraction", wherein the RBM3 protein expression of only the cytoplasms of the cells is taken into account; or a "nuclear fraction", wherein the RBM3 protein expression of only the nuclei of the cells is taken into account. The nuclear fraction may for example be classified as < 2 %, 2 - 25 %, > 25 - 50 %, > 50 - 75 % or > 75 % immunoreactive cells of the relevant cell population. The "nuclear fraction" corresponds to the percentage of relevant cells in a sample that exhibits a positive staining in the nucleus, wherein a medium or distinct and strong immunoreactivity in the nucleus is considered positive and no or faint immunoreactivity in the nucleus is considered negative. The person skilled in the art of pathology understands which cells that are relevant under the conditions present when performing the method and may determine a nuclear fraction based on his general knowledge and the teachings of the present disclosure. The relevant cells may for example be tumor cells. Further, the skilled artisan understands how to perform corresponding measurements employing the "cytoplasmic fraction" or "cellular fraction".

Another alternative for the quantification of RBM3 protein expression in a sample, such as the sample earlier obtained from the subject or the reference sample, is the determination of the overall staining intensity of the sample. The intensity may for example be: a "cellular intensity", wherein the RBM3 protein expression of the whole cells is taken into account; a "cytoplasmic intensity", wherein the RBM3 protein expression of only the cytoplasms of the cells is taken into account, or a "nuclear intensity", wherein the RBM3 protein expression of only the nuclei of the cells is taken into account. Nuclear intensity is subjectively evaluated in accordance with standards used in clinical histopathological diagnostics. Outcome of a nuclear intensity determination may be classified as: absent = no overall immunoreactivity in the nuclei of relevant cells of the sample, weak = faint overall immunoreactivity in the nuclei of relevant cells of the sample, moderate = medium overall immunoreactivity in the nuclei of relevant cells of the sample, or strong = distinct and strong overall immunoreactivity in the nuclei of relevant cells of the sample. The person skilled in the art understands which cells that are relevant under the conditions present when performing the method and may determine a nuclear intensity based on his general knowledge and the teachings of the present disclosure. The relevant cells may for example be tumor cells. Further, the skilled artisan understands how to perform corresponding measurements employing the "cytoplasmic intensity" or "cellular intensity".

The inventors have found that nuclear expression of RBM3 protein is particularly relevant for establishing prognoses.

Thus, in embodiments of the methods of the above aspects, the reference value may be a nuclear fraction, a nuclear intensity or a combination thereof. Accordingly, the sample value may be a nuclear fraction, a nuclear intensity or a combination thereof.

Thus, in embodiments of the methods of the above aspects, the reference value of step b) is a nuclear fraction of 95 % or lower, such as 90 % or lower, such as 85 % or lower, such as 80 % or lower, such as 75 % or lower, such as 70 % or lower, such as 65 % or lower, such as 60 % or lower, such as 55 % or lower, such as 50 % or lower, such as 45 % or lower, such as 40 % or lower, such as 35 % or lower, such as 30 % or lower, such as 25 % or lower, such as 20 % or lower, such as 15 % or lower, such as 10 % or lower, such as 5 % or lower, such as 2 % or lower, such as 1 % or lower, such as 0 %.

Further, in embodiments of the methods of the above aspects the reference value of step b) may be a moderate nuclear intensity of RBM3 protein expression or lower, such as a weak nuclear intensity of RBM3 protein expression or lower, such as an absent nuclear of RBM3 protein expression.

A reference value in the lower range has been found to be particularly preferred. As an example, a nuclear score (NS) of 2 was employed as the reference value (see the Example for the calculation of the NS) in Examples, section 1, below. Thus, the reference value may preferably be a nuclear fraction of 0-50 %, such as 0-25 %, an absent or weak nuclear intensity or a function of nuclear fraction and a nuclear intensity in theses ranges.

In embodiments of the methods of the above aspects, the reference value may thus be a combination or a function of a fraction value and an intensity value (see e.g. the NS employed in Examples, section 1). The reference value may thus involve two, and even more, criteria.

In general, the selection of an intensity value and/or a fraction value as the reference value may depend on the staining procedure, e.g., on the type and amount/concentration of the employed antibody and on the type and concentration of the staining reagents.

Guided by the present disclosure, a person skilled in the art, e.g., a pathologist understands how to perform the evaluation yielding a fraction, such as a cellular, cytoplasmic or nuclear fraction, or an intensity, such as a cellular, cytoplasmic or nuclear intensity. For example, the skilled artisan may use a reference sample comprising a predetermined amount of RBM3 protein for establishing the appearance of a certain fraction or intensity.

However, a reference sample may not only be used for the provision of the actual reference value, but also for the provision of an example of a sample with an amount of RBM3 that is higher than the amount corresponding to the reference value. As an example, in histochemical staining, such as in immunohistochemical staining, the skilled artisan may use a reference sample for establishing the appearance of a stained sample having a high amount of RBM3 protein, e.g., a positive reference. Subsequently, the skilled artisan may assess the appearances of samples having lower amounts of RBM3 protein, such as the appearance of a sample with an amount of RBM3 protein corresponding to the reference value. In other words, the skilled artisan may use a reference sample to create a mental image of a reference value corresponding to an amount of RBM3 protein which is lower than that of the reference sample. Alternatively, or as a complement, in such assessments, the skilled artisan may use another reference sample having a low amount of RBM3 protein, or lacking detectable RBM3 protein, for establishing the appearance of such sample, e.g., as a "negative reference".

For example, if a weak nuclear intensity is used as the reference value, two reference samples may be employed: a first reference sample having no detectable RBM3 protein, and thus corresponding to an absent nuclear intensity, which is lower than the reference value; and a second reference sample having an amount of RBM3 protein corresponding to a strong nuclear intensity, which is higher than the reference value.

Consequently, in the evaluation, the skilled artisan may use a reference sample for establishing the appearance of a sample with a high amount of RBM3 protein. Such reference sample may be a sample comprising tissue expressing a high amount of RBM3 protein, such as a sample comprising prostate cancer tissue having a pre-established high expression of RBM3 protein.

Accordingly, the reference sample may provide an example of a strong nuclear intensity (NI). With the knowledge of the appearance of a sample with strong NI, the skilled artisan may then divide samples into the NI categories absent, weak, moderate and strong. This division may be further assisted by a reference sample lacking detectable RBM3 protein (negative reference), i.e., a reference sample providing an absent nuclear intensity. Also, the reference sample may provide an example of a sample with a nuclear fraction (NF) higher than 75 %. With the knowledge of the appearance of a sample with more than 75 % positive cells, the skilled artisan may then evaluate the NF of other samples having e.g., a lower percentage of positive cells. This division may be further assisted by a reference sample essentially lacking RBM3 protein (negative reference), i.e., a reference sample providing a low NF (e.g., < 5%, such as < 2%), or a NF of 0.

As mentioned above, cell lines expressing a controlled amount of RBM3 protein may be used as the reference, in particular as a positive reference.

One or more pictures may also be provided as the "reference sample". For example, such a picture may show an example of a tumor tissue slide stained with a certain antibody during certain conditions and exhibiting a certain nuclear intensity and/or fraction. The above discussion about the "reference sample" applies *mutatis mutandis* to pictures.

In some embodiments, step a) of the methods of the above aspects may comprise:
obtaining biological material from the subject, excising or selecting a relevant part of the biological material to obtain said sample and optionally arranging the sample on a solid phase to facilitate the evaluation of step a). Step a) may thus, as an example, comprise obtaining prostate tumor tissue material from the subject, optionally fixating the tissue material in paraffin or formalin, histo-processing the tissue material to obtain a section which constitute said sample and optionally mounting said sample on a transparent slide, such as a glass slide, for microscopy.

In embodiments of the methods of the aspects above, the RBM3 protein may be detected and/or quantified through the application to the sample of a detectable and/or quantifiable affinity ligand, which is capable of selective interaction with the RBM3 protein. The application of the affinity ligand is performed under conditions that enable binding of the affinity ligand to RBM3 protein in the sample.

To concretize, in embodiments of the methods of the aspects above, step a) may comprise:
a1) applying to said sample a quantifiable affinity ligand capable of selective interaction with the RBM3 protein to be evaluated, said application being performed under conditions that enable binding of said affinity ligand to RBM3 protein present in said sample;
a2) removing non-bound affinity ligand; and
a3) quantifying the affinity ligand remaining in association with said sample to evaluate said amount.

"Affinity ligand remaining in association with the sample" refers to affinity ligand which was not removed in step a2), e.g., the affinity ligand bound to the sample. Here, the binding may for example be the interaction between antibody and antigen.

However, in some embodiments, the removal of non-bound affinity ligand according to a2), e.g. the washing, is not always necessary. Thus, in some embodiments of the methods of the aspects above, step a) may comprise:
al) applying to said sample a quantifiable affinity ligand capable of selective interaction with the RBM3 protein to be evaluated, said application being performed under conditions that enable binding of said affinity ligand to RBM3 protein present in said sample;
all) quantifying the affinity bound to said sample to evaluate said amount.

In the context of the present disclosure, "specific" or "selective" interaction of e.g., an affinity ligand with its target or antigen means that the interaction is such that a distinction between specific and non-specific, or between selective and non-selective, interaction becomes meaningful. The interaction between two proteins is sometimes measured by the dissociation constant. The dissociation constant describes the strength of binding (or affinity) between two molecules. Typically the dissociation constant between an antibody and its antigen is from 10⁻⁷ to 10⁻¹¹ M. However, high specificity/selectivity does not necessarily require high affinity. Molecules with low affinity (in the molar range) for its counterpart have been shown to be as selective/specific as molecules with much higher affinity. In the case of the present disclosure, a specific or selective interaction refers to the extent to which a particular method can be used to determine the presence and/or amount of a specific protein, the target protein, under given conditions in the presence of other proteins in a biological sample, such as a tissue sample or a fluid sample of a naturally occurring or processed biological fluid. In other words, specificity or selectivity is the capacity to distinguish between related proteins. For example, the specificity or selectivity of an antibody may be determined as in WO 2010/092190 (see Examples, Section 2), wherein analysis is performed using a protein array set-up, a suspension bead array and a multiplexed competition assay, respectively. Specificity and selectivity determinations are also described in Nilsson P et al. (2005) Proteomics 5:4327-4337.

It is regarded as within the capabilities of those of ordinary skill in the art to select or manufacture the proper affinity ligand and to select the proper format and conditions for detection and/or quantification. Nevertheless, examples of affinity ligands that may prove useful, as well as examples of formats and conditions for detection and/or quantification, are given below.

Thus, in embodiments of the present disclosure, the affinity ligand may be selected from the group consisting of antibodies, fragments thereof and derivatives thereof, i.e., affinity ligands based on an immunoglobulin scaffold. The antibodies and the fragments or derivatives thereof may be isolated. Antibodies comprise monoclonal and polyclonal antibodies of any origin, including murine, rabbit, human and other antibodies, as well as chimeric antibodies comprising sequences from different species, such as partly humanized antibodies, e.g., partly humanized mouse antibodies. Polyclonal antibodies are produced by immunization of animals with the antigen of choice. The polyclonal antibodies may be antigen purified. Monoclonal antibodies of defined specificity can be produced using the hybridoma technology developed by Köhler and Milstein (Köhler G and Milstein C (1976) Eur. J. Immunol. 6:511-519). The antibody fragments and derivatives of the present disclosure are capable of selective interaction with the same antigen (e.g. RBM3 protein) as the antibody they are fragments or derivatives of. Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137); Bence Jones dimers (Stevens FJ et al. (1991) Biochemistry 30:6803-6805); camelid heavy-chain dimers (Hamers-Casterman C et al. (1993) Nature 363:446-448) and single variable domains (Cai X and Garen A (1996) Proc. Natl. Acad. Sci. U.S.A. 93:6280-6285; Masat L et al. (1994) Proc. Natl. Acad. Sci. U.S.A. 91:893-896), and single domain scaffolds like e.g., the New Antigen Receptor (NAR) from the nurse shark (Dooley H et al. (2003) Mol. Immunol. 40:25-33) and minibodies based on a variable heavy domain (Skerra A and Plückthun A (1988) Science 240:1038-1041).

In some embodiments, the affinity ligand of the present disclosure is capable of selective interaction with a peptide consisting of the amino acid sequence SEQ ID NO:1. The RBM3 fragment SEQ ID NO:1 was designed to lack transmembrane regions to ensure efficient expression in *E. coli*, and to lack any signal peptide, since those are cleaved off in the mature protein. SEQ ID NO:1 was thus designed for immunizations. In addition, the protein fragment was designed to consist of a unique sequence with low sequence identity to other human proteins, to minimize cross reactivity of generated affinity reagents, and to be of a suitable size to allow the formation of conformational epitopes and still allow efficient cloning and expression in bacterial systems. Accordingly, in the cases wherein the affinity ligand is an antibody or fragment o derivative thereof, the affinity ligand may be obtainable by a process comprising a step of immunizing an animal with a peptide whose amino acid sequence consists of the sequence SEQ ID NO:1. For example, an immunization process may comprise primary immunization with the protein/antigen in Freund's complete adjuvant. Also, the immunization process may further comprise boosting at least two times, in intervals of 2-6 weeks, with the protein/antigen in Freund's incomplete adjuvant. Processes for the production of antibodies or fragments or derivatives thereof against a given target are known in the art.

In the context of the present disclosure, an "antigen purified antibody" is one or a population of polyclonal antibodies which has been affinity purified on its own antigen, thereby separating such antigen purified antibodies from other antiserum proteins and non-specific antibodies. This affinity purification results in antibodies that bind selectively to its antigen. In the case of the present disclosure, the polyclonal antisera are purified by a two-step immunoaffinity based protocol to obtain antigen purified antibodies selective for the target protein. Antibodies directed against generic affinity tags of antigen fragments are removed in a primary depletion step, using the immobilized tag protein as the capturing agent. Following the first depletion step, the serum is loaded on a second affinity column with the antigen as capturing agent, in order to enrich for antibodies specific for the antigen (see also Nilsson P et al. (2005) Proteomics 5:4327-4337).

Polyclonal and monoclonal antibodies, as well as their fragments and derivatives, represent the traditional choice of affinity ligands in applications requiring selective biomolecular recognition, such as in the detection and/or quantification of RBM3 protein according to the method aspects above. However, those of skill in the art know that, due to the increasing demand of high throughput generation of selective binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific/selective affinity ligands are then selected using a suitable selection platform. The scaffold molecule may be of immunoglobulin protein origin (Bradbury AR and Marks JD (2004) J. Immunol. Meths. 290:29-49), of non-immunoglobulin protein origin (Nygren PÅ and Skerra A (2004) J. Immunol. Meths. 290:3-28), or of an oligonucleotide origin (Gold L et al. (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against RBM3 protein for use according to the present disclosure, are staphylococcal protein A and domains thereof and derivatives of these domains, such as protein Z (Nord K et al. (1997) Nat. Biotechnol. 15:772-777); lipocalins (Beste G et al. (1999) Proc. Natl. Acad. Sci. U.S.A. 96:1898-1903); ankyrin repeat domains (Binz HK et al. (2003) J. Mol. Biol. 332:489-503); cellulose binding domains (CBD) (Smith GP et al. (1998) J. Mol. Biol. 277:317-332; Lehtiö J et al. (2000) Proteins 41:316-322); γ crystallines (Fiedler U and Rudolph R, WO01/04144); green fluorescent protein (GFP) (Peelle B et al. (2001) Chem. Biol. 8:521-534); human cytotoxic T lymphocyte-associated antigen 4 (CTLA-4) (Hufton SE et al. (2000) FEBS Lett. 475:225-231; Irving RA et al. (2001) J. Immunol. Meth. 248:31-45); protease inhibitors, such as Knottin proteins (Wentzel A et al. (2001) J. Bacteriol. 183:7273-7284; Baggio R et al. (2002) J. Mol. Recognit. 15:126-134) and Kunitz domains (Roberts BL et al. (1992) Gene 121:9-15; Dennis MS and Lazarus RA (1994) J. Biol. Chem. 269:22137-22144); PDZ domains (Schneider S et al. (1999) Nat. Biotechnol. 17:170-175); peptide aptamers, such as thioredoxin (Lu Z et al. (1995) Biotechnology 13:366-372; Klevenz B et al. (2002) Cell. Mol. Life Sci. 59:1993-1998); staphylococcal nuclease (Norman TC et al. (1999) Science 285:591-595); tendamistats (McConell SJ and Hoess RH (1995) J. Mol. Biol. 250:460-479; Li R et al. (2003) Protein Eng. 16:65-72); trinectins based on the fibronectin type III domain (Koide A et al. (1998) J. Mol. Biol. 284:1141-1151; Xu L et al. (2002) Chem. Biol. 9:933-942); and zinc fingers (Bianchi E et al. (1995) J. Mol. Biol. 247:154-160; Klug A (1999) J. Mol. Biol. 293:215-218; Segal DJ et al. (2003) Biochemistry 42:2137-2148).

The above-mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two-hybrid system (Fields S and Song O (1989) Nature 340:245-246), yeast display (Gai SA and Wittrup KD (2007) Curr Opin Struct Biol 17:467-473), mRNA display (Roberts RW and Szostak JW (1997) Proc. Natl. Acad. Sci. U.S.A. 94:12297-12302), bacterial display (Daugherty PS (2007) Curr Opin Struct Biol 17:474-480, Kronqvist N et al. (2008) Protein Eng Des Sel 1-9, Harvey BR et al. (2004) PNAS 101 (25):913-9198), microbead display (Nord O et al. (2003) J Biotechnol 106:1-13, WO01/05808), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the present disclosure, the affinity ligand may be a non-immunoglobulin affinity ligand derived from any of the protein scaffolds listed above, or an oligonucleotide molecule.

As mentioned above, the RBM3 protein fragment SEQ ID NO:1 was designed to consist of a unique sequence with low sequence identity to other human proteins and to minimize cross reactivity of generated affinity reagents. Consequently, in embodiments of the present disclosure, the affinity ligand may be capable of selective interaction with a polypeptide consisting of the amino acid sequence SEQ ID NO:1.

The epitope regions SEQ ID NO:4 and 5 has been identified within SEQ ID NO:1. Thus, in some embodiments, the affinity ligand of the present disclosure is capable of selective interaction with a peptide consisting of an amino acid sequence selected from SEQ ID NO:4 and 5.

Further, another four epitope regions (SEQ ID NO:6-9) have been identified. Thus, in some embodiments, the affinity ligand of the present disclosure is capable of selective interaction with an RBM3 fragment which consists of 20 amino acid residues or less, such as 15 amino acid residues or less, and comprises a sequence selected from SEQ ID NO:6-9.

Also, another ten epitope regions (SEQ ID NO:10-19) have been identified. Thus, in some embodiments, the affinity ligand of the present disclosure is capable of selective interaction with an RBM3 fragment which consists of 20 amino acid residues or less, such as 15 amino acid residues or less, and comprises a sequence selected from SEQ ID NO:10-19.

Antibodies having selectivity for a single epitope region (such as monoclonal antibodies) may provide for increased reproducibility in detection analyses as compared to antibodies generated against a longer peptide sequence (such as a PrEST or a full-length protein). The antibodies selective for a single epitope region may also provide for distinct and strong staining in immunohistochemical analyses. These benefits, independently or jointly, may be valuable when and making treatment predictions or decisions regarding treatments according to the present disclosure.

The monoclonal antibodies named "6F11" and "1 B5" are considered to be particularly beneficial. 6F11 and 1 B5 have both been shown to be more selective than a polyclonal anti-RBM3 antibody. Further, 1 B5 has been shown to be more selective than 6F11. 1 B5 is also employed in the example section below.

SEQ ID NO:17, to which 1 B5 binds, is within SEQ ID NO:5. In preferred embodiments of the present disclosure, the affinity ligand is thus capable of selective interaction with an RBM3 fragment which consists of SEQ ID NO:5, and in particularly preferred embodiments of the present disclosure, the affinity ligand is capable of selective interaction with an RBM3 fragment which consists of 20 amino acid residues or less, such as 15 amino acid residues or less, and comprises the sequence SEQ ID NO:17.
6F11 has been shown to bind to SEQ ID NO:8 and SEQ ID NO:16. In other preferred embodiments of the present disclosure, the affinity ligand is thus capable of selective interaction with an RBM3 fragment which consists of 20 amino acid residues or less, such as 15 amino acid residues or less, and comprises a sequence selected from SEQ ID NO:8 and 16. Note that SEQ ID NO:8 and 16 are overlapping and that such a fragment may comprise the sequences of both SEQ ID NO:8 and 16.

In Examples below, antibodies capable of selective interaction with SEQ ID NO:24 and 25 (both within SEQ ID NO:4) are shown to be particularly selective. In preferred embodiments of the present disclosure, the affinity ligand is thus capable of selective interaction with an RBM3 fragment which consists of a sequence selected from SEQ ID NO:24 and 25.

Further, the epitope regions SEQ ID NO:22 and 26 have been identified. In other preferred embodiments of the present disclosure, the affinity ligand is thus capable of selective interaction with an RBM3 fragment which comprises a sequence selected from SEQ ID NO:22 and 26 and consists of 21 amino acids or less, such as 20 amino acids or less, such as 17 amino acids or less, such as 15 amino acid residues or less, such as 10 amino acid residues or less, such as 6 amino acids or less.

The detection and/or quantification of the affinity ligand capable of selective interaction with the RBM3 protein may be accomplished in any way known to the skilled person for detection and/or quantification of binding reagents in assays based on biological interactions. Accordingly, any affinity ligand described above may be used to quantitatively and/or qualitatively detect the presence of the RBM3 protein. These "primary" affinity ligands may be labeled themselves with various markers or may in turn be detected by secondary, labeled affinity ligands to allow detection, visualization and/or quantification. This can be accomplished using any one or more of a multitude of labels, which can be conjugated to the affinity ligand capable of interaction with RBM3 protein or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g., fluorescein, rhodamine, phycoerythrin, fluorescamine), chromophoric dyes (e.g., rhodopsin), chemiluminescent compounds (e.g., luminal, imidazole) and bioluminescent proteins (e.g., luciferin, luciferase), haptens (e.g., biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g., horseradish peroxidase, alkaline phosphatase, beta-lactamase), radioisotopes (e.g., ³H, ¹⁴C, ³²P, ³⁵S or ¹²⁵I) and particles (e.g., gold). In the context of the present disclosure, "particles" refer to particles, such as metal particles, suitable for labeling of molecules. Such particles are well known to the skilled person. Further, the affinity ligands may also be labeled with fluorescent semiconductor nanocrystals (quantum dots). Quantum dots have superior quantum yield and are more photostable compared to organic fluorophores and are therefore more easily detected (Chan et al. (2002) Curr Opi Biotech. 13: 40-46). The different types of labels can be conjugated to an affinity ligand using various chemistries, e.g., the amine reaction or the thiol reaction. However, other reactive groups than amines and thiols can be used, e.g., aldehydes, carboxylic acids and glutamine.

The method aspects above may be put to use in any of several known formats and set-ups, of which a non-limiting selection is discussed below.

In a set-up based on histology, the detection, localization and/or quantification of a labeled affinity ligand bound to its RBM3 protein target may involve visualizing techniques, such as light microscopy or immunofluoresence microscopy. Other methods may involve the detection via flow cytometry or luminometry.

Biological material from the subject may be used for obtaining the sample for detection and/or quantification of RBM3 protein. The sample may thus be an earlier obtained sample. If using an earlier obtained sample in a method, no steps of the method are practiced on the human or animal body.

The affinity ligand may be applied to the sample for detection and/or quantification of the RBM3 protein. This procedure enables not only detection of RBM3 protein, but may in addition show the distribution and relative level of expression thereof.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light in a specific wavelength region. The presence of a luminescently tagged affinity ligand may be detected and/or quantified by luminescence developed during a chemical reaction. Detection of an enzymatic reaction is due to a color shift in the sample arising from a chemical reaction. Those of skill in the art are aware that a variety of different protocols can be modified in order for proper detection and/or quantification.

In embodiments of the methods of the above aspects, the sample may be immobilized onto a solid phase support or carrier, such as nitrocellulose or any other solid support matrix capable of immobilizing RBM3 protein present in the biological sample applied to it. Some well-known solid state support materials useful in the present invention include glass, carbohydrate (e.g., Sepharose), nylon, plastic, wool, polystyrene, polyethene, polypropylene, dextran, amylase, films, resins, cellulose, polyacrylamide, agarose, alumina, gabbros and magnetite. After immobilization of the biological sample, primary affinity ligand selective for RBM3 protein may be applied. If the primary affinity ligand is not labeled in itself, the supporting matrix may be washed with one or more appropriate buffers known in the art, followed by exposure to a secondary labeled affinity ligand and washed once again with buffers to remove unbound affinity ligands. Thereafter, selective affinity ligands may be detected and/or quantified with conventional methods. The binding properties for an affinity ligand may vary from one solid state support to the other, but those skilled in the art should be able to determine operative and optimal assay conditions for each determination by routine experimentation.

Consequently, in embodiments of the methods of the above aspects, the quantifiable affinity ligand of a1) or al) may be detected using a secondary affinity ligand capable of recognizing the quantifiable affinity ligand. The quantification of a3) or all) may thus be carried out by means of a secondary affinity ligand with affinity for the quantifiable affinity ligand. As an example, the secondary affinity ligand may be an antibody or a fragment or a derivative thereof.

As an example, one available method for detection and/or quantification of the RBM3 protein is by linking the affinity ligand to an enzyme that can then later be detected and/or quantified in an enzyme immunoassay (such as an EIA or ELISA). Such techniques are well established, and their realization does not present any undue difficulties to the skilled person. In such methods, the biological sample is brought into contact with a solid material or with a solid material conjugated to an affinity ligand against the RBM3 protein, which is then detected and/or quantified with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the present disclosure are ³H, ¹⁴C, ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize RBM3 protein by detection of radioactivity *in vivo* or *ex vivo.* Radionuclear scanning with e.g., gamma camera, magnetic resonance spectroscopy or emission tomography function for detection *in vivo* and ex *vivo,* while gamma/beta counters, scintillation counters and radiographies are also used *ex vivo.*

Methods for detecting and quantifying biomarkers on the mRNA level are well known within the art.

According to one such method, total cellular RNA is purified from cells by homogenization in the presence of nucleic acid extraction buffer, followed by centrifugation. Nucleic acids are then precipitated, in order to remove DNA by treatment with DNase and precipitation. The RNA molecules are then separated by gel electrophoresis on agarose gels according to standard techniques, and transferred to nitrocellulose filters by, e.g., the so-called "Northern" blotting technique. The RNA is then immobilized on the filters by heating. Detection and quantification of specific RNA is accomplished using appropriately labeled DNA or RNA probes complementary to the RNA in question. See, for example, Molecular Cloning: A Laboratory Manual (Sambrook J.et al., (1989) 2nd edition, Cold Spring Harbor Laboratory Press). Methods for the preparation of labeled DNA and RNA probes, and the conditions for hybridization thereof to target nucleotide sequences, are described in Molecular Cloning: A Laboratory Manual (Sambrook J.et al., (1989) 2nd edition, Cold Spring Harbor Laboratory Press). For example, the nucleic acid probe may be labeled with, e.g., a radionuclide such as ³H, ³²P, ³³P, ¹⁴C, or ³⁵S; a heavy metal; or a ligand capable of functioning as a specific binding pair member for a labeled ligand (e.g., biotin, avidin, or an antibody), a fluorescent molecule, a chemi luminescent molecule, an enzyme, or the like.

Probes may be labeled to high specific activity by either the nick translation method (Rigby et al., (1977) J. Mol Biol, 113: 237-251), or by the random priming method (Fienberg, (1983) Anal. Biochem., 132: 6-13). The latter can be a method for synthesizing ³²P-labeled probes of high specific activity from RNA templates. For example, by replacing preexisting nucleotides with highly radioactive nucleotides according to the nick translation method, it is possible to prepare ³²P- labeled nucleic acid probes with a specific activity well in excess of 10 cpm/microgram. Autoradiographic detection of hybridization then can be performed by exposing hybridized filters to photographic film. Densitometric scanning of the photographic films exposed by the hybridized filters provides an accurate measurement of biomarker levels. Using another approach, biomarker levels can be quantified by computerized imaging systems, such as the Molecular Dynamics 400-B 2D Phosphorimager (Amersham Biosciences, Piscataway, NJ., USA).

Where radionuclide labeling of DNA or RNA probes is not practical, the random- primer method can be used to incorporate an analogue, for example, the dTTP analogue 5 -(N-(N- biotinyl-epsilon-aminocaproyl)-3-aminoallyl)deoxyuridine triphosphate, into the probe molecule. The biotinylated probe oligonucleotide can be detected by reaction with biotin-binding proteins, such as avidin, streptavidin, and antibodies (e.g., anti-biotin antibodies) coupled to fluorescent dyes or enzymes that produce color reactions.

In addition to Northern and other RNA blotting hybridization techniques, determining the levels of RNA transcript may be accomplished using the technique of in situ hybridization. This technique requires fewer cells than the Northern blotting technique, and involves depositing whole cells onto a microscope cover slip and probing the nucleic acid content of the cell with a solution containing radioactive or otherwise labeled nucleic acid (e.g., cDNA or RNA) probes. This technique is particularly well-suited for analyzing tissue biopsy samples from subjects.

The relative number of RNA transcripts in cells also can be determined by reverse transcription of RNA transcripts, followed by amplification of the reverse-transcribed transcripts by polymerase chain reaction (RT-PCR). The levels of RNA transcripts can be quantified in comparison with an internal standard, for example, the level of mRNA from a standard gene present in the same sample. The person skilled in the art is capable of selecting suitable genes for use as an internal standard. The methods for quantitative RT-PCR and variations thereof are within the skill in the art.

Any suitable primers can be used for the quantitative RT-PCR. Preferably, the primers are specific to RBM3. It is within the skill in the art to generate primers specific to RBM3 (e.g. starting from SEQ ID NO:3). Primers can be of any suitable length, but are preferably between 19 and 23 (e.g., 19, 20, 21, 22, or 23) nucleotides. Ideally, amplicon length should be 50 to 150 (up to 250 may be necessary but then optimization of the thermal cycling protocol and reaction components may be necessary) bases for optimal PCR efficiency. Designing primers that generate a very long amplicon may lead to poor amplification efficiency. Information about primer design and optimal amplicon size may fo example be found at www.ambion.com.

In some instances, it may be desirable to use microchip technology to detect biomarker expression. The microchip can be fabricated by techniques known in the art. For example, probe oligonucleotides of an appropriate length, e.g., 40 nucleotides, are 5'-amine modified at position C6 and printed using commercially available microarray systems, e.g., the GENEMACHINE OmniGrid 100 Microarrayer and Amersham CODELINK activated slides. Labeled cDNA oligomer corresponding to the target RNAs is prepared by reverse transcribing the target RNA with labeled primer. Following first strand synthesis, the RNA/DNA hybrids are denatured to degrade the RNA templates. The labeled target cDNAs thus prepared are then hybridized to the microarray chip under hybridizing conditions, e.g., 6 times SSPE/30% formamide at 25 °C for 18 hours, followed by washing in 0.75 times TNT at 37 °C for 40 minutes. At positions on the array, where the immobilized probe DNA recognizes a complementary target cDNA in the sample, hybridization occurs. The labeled target cDNA marks the exact position on the array where binding occurs, thereby allowing automatic detection and quantification. The output consists of a list of hybridization events, which indicate the relative abundance of specific cDNA sequences, and therefore the relative abundance of the corresponding complementary biomarker, in the subject sample. According to one embodiment, the labeled cDNA oligomer is a biotin-labeled cDNA prepared from a biotin-labeled primer. The microarray is then processed by direct detection of the biotin-containing transcripts using, e.g., Streptavidin-Alexa647 conjugate, and scanned utilizing conventional scanning methods. Image intensities of each spot on the array are proportional to the abundance of the corresponding biomarker in the subject sample.

The use of the array has one or more advantages for mRNA expression detection. First, the global expression of several to thousands of genes can be identified in a single sample at one time. Second, through careful design of the oligonucleotide probes, the expression of both mature and precursor molecules can be identified. Third, in comparison with Northern blot analysis, the chip requires a small amount of RNA.

The RBM3 mRNA may for example be extracted from formalin-fixed, paraffin-embedded tumor tissue. Accordingly, the sample of the methods of the present disclosure may be formalin-fixed and/or paraffin-embedded prostate tumor tissue independent of if RBM3 protein or RBM3 m RNA is detected.

The inventors have realized that RBM3 mRNA analysis of the present disclosure may be incorporated in an mRNA-based assay designed to support individualized treatment planning. Such an assay may employ RT-PCR to analyze the expression of several genes.

Following the findings presented above, the inventors have realized several uses for the RBM3 protein and fragments thereof.

As a fifth aspect of the present disclosure, there is provided a use of an RBM3 protein as a prognostic marker for prostate cancer. The protein is preferably provided in a sample, such as a prostate tissue sample, from a subject having a prostate cancer.

Normally, the RBM3 protein of the fifth aspect will be used as a marker of a relatively good prostate cancer prognosis.

In a preferred embodiment, the use is as a stage-independent prognostic marker for prostate cancer.

The use of the fifth aspect may be entirely *ex vivo.*

In the context of the present disclosure, "prognostic marker" refers to something material which presence indicates a prognosis. The marker may thus be a biomarker, such as a human protein.

As a sixth aspect or the present disclosure, there is provided a use of an RBM3 protein, or an antigenically active fragment thereof, for the production, selection or purification of a prognostic agent for a mammalian subject having a prostate cancer.

In the context of the present disclosure, an "antigenically active fragment" of an RBM3 protein is a fragment of sufficient size to be useful for the generation of an affinity ligand, e.g., an antibody, which selectively interacts with an RBM3 protein comprising the fragment.

The selection and purification may be *ex vivo,* while the production may be *in vivo* (in an animal).

In the context of the present disclosure, "prognostic agent" refers to an agent having at least one property being valuable in an establishment of a prognosis, e.g., a prognosis for a mammalian subject having a prostate cancer. For example, the prognostic agent may be capable of selective interaction with the prognostic marker.

The prognostic agent may be an affinity ligand capable of selective interaction with the RBM3 protein or the antigenically active fragment thereof. Examples of such affinity ligands are discussed above in connection with the method aspects.

Guided by the teachings of the present disclosure, the person skilled in the art understands how to use the RBM3 protein or fragment in the production, selection or purification of the prognostic agent. For example, the use may comprise affinity purification on a solid support onto which the RBM3 protein has been immobilized. The solid support may for example be arranged in a column. Further, the use may comprise selection of affinity ligands having specificity for the RBM3 protein using a solid support onto which the polypeptide has been immobilized. Such solid support may be well plates (such as 96 well plates), magnetic beads, agarose beads or sepharose beads. Further, the use may comprise analysis of affinity ligands on a soluble matrix, for example using a dextran matrix, or use in a surface plasmon resonance instrument, such as a Biacore™ instrument, wherein the analysis may for example comprise monitoring the affinity for the immobilized RBM3 protein of a number of potential affinity ligands.

Also, for the production of the prognostic agent, the RBM3 protein or an antigenically active fragment thereof may be used in an immunization of an animal, such as a rabbit or mouse.

Such use may be involved in a method comprising the steps:
i) immunizing an animal using the RBM3 protein or antigenically an active fragment thereof as the antigen;
ii) obtaining serum comprising the prognostic agent from the immunized animal; and, optionally,
iii) isolating the prognostic agent from the serum.

Alternatively the steps following the first step may be:
ii') obtaining cells from the immunized animal, which cells comprise DNA encoding the prognostic agent,
iii') fusing the cells with myeloma cells to obtain at least one clone, and
iv') obtaining the prognostic agent expressed by the clone.

In embodiments of the fifth or sixth aspect, the amino acid sequence of the RBM3 protein may comprise a sequence selected from:
i) SEQ ID NO:1; and
ii) a sequence which is at least 85 % identical to SEQ ID NO:1.

In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1.

Further, in embodiments of the fifth or sixth aspect the amino acid sequence of the RBM3 protein may comprise or consist of a sequence selected from:
i) SEQ ID NO:2; and
ii) a sequence which is at least 85 % identical to SEQ ID NO:2.

In some embodiments, sequence ii) is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:2.

The antigenically active fragment of the sixth aspect may for example be an RBM3 protein fragment which consists of 50 amino acid residues or less and comprises a sequence selected from SEQ ID NO:4-19 and 22-26. In embodiments of the sixth aspect, the fragment consists of 29 amino acid residues or less. In further embodiments of the sixth aspect, the fragment consists of 21 amino acid residues or less, such as 17 amino acids or less, such as 15 amino acid residues or less, and comprises a sequence selected from SEQ ID NO:6-19 and 22-26.

As a seventh aspect of the present disclosure, there is provided a RBM3 protein fragment (as such) consisting of 17 amino acid residues or less and comprising a sequence selected from SEQ ID NO: 22, 24, 25 and 26. As an example, the RBM3 protein fragment may consist of 15 amino acids residues or less. As a further example, the RBM3 protein fragment may consist of 10 amino acid residues or less, such as 6 amino acids or less, and comprise a sequence selected from SEQ ID NO: 22 and 26.

Various applications of the fragment of the seventh aspect are described above in connection with the sixth aspect. Also, the fragment of the sixth aspect is shown to provide for high selectivity in the Examples below.

Different embodiments of an affinity ligand capable of selective interaction with a RBM3 protein or a fragment thereof are discussed above in connection with the method aspects.

As an eighth aspect of the present disclosure, there is provided a use of such an affinity ligand as a prostate cancer prognostic agent. Consequently, the affinity ligand may be used for establishing a prognosis for a subject having a prostate cancer. Such use may for example be performed ex *vivo,* e.g., involving the determination of the amount of RBM3 in at least part of a sample earlier obtained from the subject. In a preferred embodiment, the use is as a stage-independent prognostic agent for prostate cancer.

As a ninth aspect of the present disclosure, there is provided an affinity ligand (as such) capable of selective interaction with an RBM3 protein fragment consisting of 21 amino acid residues or less and comprising a sequence selected from SEQ ID NO: 22-26. Such affinity ligands (in particular monoclonal antibodies) are described in Examples below. As an example, the affinity ligand is capable of selective interaction with a RBM3 protein fragment consisting of 17 amino acid residues or less, such as 15 amino acid residues or less, and comprising a sequence selected from SEQ ID NO: 22, 24, 25 and 26. As a further example, the affinity ligand is capable of selective interaction with a RBM3 protein fragment consisting of 10 amino acid residues or less and comprising a sequence selected from SEQ ID NO: 22 and 26. Also, the affinity ligand may be capable of selective interaction with a RBM3 protein fragment consisting of 6 amino acid residues or less and comprising the sequence SEQ ID NO: 22.

The affinity ligand may for example be provided as a staining solution in a container adapted for an automatic staining apparatus, such as BenchMark XT or BenchMark Ultra marketed by Ventana. The container may thus be connectable to an inlet on the apparatus for solutions of primary antibodies.

The affinity ligand may also be provided in a kit for carrying out a method according to the above aspects. Such a kit further comprises reagents necessary for quantifying the amount of the affinity ligand.

Various components of the kit may be selected and specified as described above in connection with the method aspects of the present disclosure.

Thus, the kit comprises an affinity ligand against an RBM3 protein, as well as other means that help to quantify the specific and/or selective affinity ligand after it has bound specifically and/or selectively to the RBM3 protein. For example, the kit may contain a secondary affinity ligand for detecting and/or quantifying a complex formed by the RBM3 protein and the affinity ligand capable of selective interaction with the RBM3 protein. The kit may also contain various auxiliary substances other than affinity ligands, to enable the kit to be used easily and efficiently. Examples of auxiliary substances include solvents for dissolving or reconstituting lyophilized protein components of the kit, wash buffers, substrates for measuring enzyme activity in cases where an enzyme is used as a label, target retrieval solution to enhance the accessibility to antigens in cases where paraffin or formalin-fixed tissue samples are used, and substances such as reaction arresters, e.g., endogenous enzyme block solution to decrease the background staining and/or counterstaining solution to increase staining contrast, that are commonly used in immunoassay reagent kits.

Further, in accordance with what is described above in connection with the method aspects, the detectable affinity ligand may in embodiments of the kit aspect comprise a label selected from the group consisting of fluorescent dyes and metals, chromophoric dyes, chemiluminescent compounds and bioluminescent proteins, enzymes, radioisotopes, particles and quantum dots. Alternatively, the reagents necessary for quantifying the amount of the affinity ligand comprise a secondary affinity ligand capable of recognizing the quantifiable affinity ligand. As an example, the secondary affinity ligand capable of recognizing the quantifiable affinity ligand comprises a label selected from the group consisting of fluorescent dyes or metals, chromophoric dyes, chemiluminescent compounds and bioluminescent proteins, enzymes, radioisotopes, particles and quantum dots.

The kit according to the kit aspect may also advantageously comprise a reference sample for provision of, or yielding, the reference value to be used for comparison with the sample value. For example, the reference sample may comprise a predetermined amount of RBM3 protein. Such a reference sample may for example be constituted by a tissue sample containing the predetermined amount of RBM3 protein. The tissue reference sample may then be used by the person of skill in the art in the determination of the RBM3 expression status in the sample being studied, by manual, such as ocular, or automated comparison of expression levels in the reference tissue sample and the subject sample. As another example, the reference sample may comprise cell lines, such as cancer cell lines, expressing a predetermined, or controlled, amount of RBM3 protein. The person skilled in the art understands how to provide such cell lines, for example guided by the disclosure of Rhodes et al. (2006) The biomedical scientist, p 515-520. As an example, the cell lines may be formalin fixed. Also, such formalin fixed cell lines may be paraffin embedded.

The wording "reference sample for provision of the reference value" is to be interpreted broadly in the context of the present disclosure. The reference sample may comprise an amount of RBM3 protein actually corresponding to the reference value, but it may also comprise an amount of RBM3 protein corresponding to a value being higher than the reference value. In the latter case, the "high" value may be used by a person performing the method as an upper reference (positive reference) for assessing, e.g., the appearance of, a reference value which is lower than the "high" value. The person skilled in the art of immunohistochemistry understands how to do such an assessment. Further, as an alternative or a complementing example, the skilled person may use another reference sample comprising a low amount of RBM3 protein for provision of a "low" value in such an assessment, e.g., as a negative reference. This is further discussed above in connection with the method aspects.

The kit may thus comprise: a reference sample comprising an amount of RBM3 protein corresponding to a predetermined reference value; a reference sample comprising an amount of RBM3 protein corresponding to a value being higher than a predetermined reference value; and/or a reference sample comprising an amount of RBM3 protein corresponding to a value being lower than or equal to a predetermined reference value.

In embodiments of the kit, the reference sample may be a tissue sample, such as a tissue sample adapted to ocular or microscopic evaluation. As an example, the tissue reference sample may be fixated in paraffin or buffered formalin and/or histo-processed to sections (e.g., µm-thin sections) that are mounted on microscopic glass-slides. The tissue reference sample may be further adapted to staining with affinity ligands, such as antibodies, against an RBM3 protein.

Consequently, the reference sample may be adapted to directly, or indirectly, provide any relevant reference value, such as any one of the reference values discussed above.

The inventors have also found that RBM3 is up-regulated in prostatic intraepithelial neoplasia (PIN), while it is absent or weakly expressed in normal prostatic glands. Accordingly, the inventors have concluded that RBM3 is a biomarker of PIN, which is a pre-stage of prostate cancer.

As an alternative aspect of the present disclosure, there is thus provided a method of determining whether a mammalian subject belongs to a first or a second group, wherein subjects of the first group have a higher risk of having a pre-stage of prostate cancer, such as prostate intraepithelial neoplasia (PIN), than subjects of the second group, comprising the steps of:
a) evaluating an amount of RBM3 in at least part of an earlier obtained sample comprising biological material from the prostate of said subject and determining a sample value corresponding to the evaluated amount;
b) comparing said sample value with a predetermined reference value;
   and
   if said sample value is higher than said reference value,
c1) concluding that the subject belongs to the first group; and if said sample value is lower than or equal to said reference value,
c2) concluding that the subject belongs to the second group.

In an embodiment of this alternative aspect, the sample is derived from prostatic fluid or prostate tissue from the subject. The tissue is preferably obtained from a biopsy taken during a prostate examination.

It may be particularly relevant to determine the risk of having a pre-stage of prostate cancer when one or more other risk indicators, such as an elevated PSA level, are present but no tumor has been found. Thus, in an embodiment of the alternative aspect, the subject has a PSA concentration of at least 10 ng/ml, such as at least 20 ng/ml. 1.

As another alternative aspect of the present disclosure, there is provided a use ex *vivo* of an RBM3 protein as a diagnostic marker for a pre-stage of prostate cancer, such as PIN. The RBM3 protein may for example be provided in a prostate tissue sample, e.g. obtained from a biopsy. Such biopsies are regularly taken in prostate examinations.

As another alternative aspect, there is provided a use of an RBM3 protein, or an antigenically active fragment thereof, for the production, selection or purification of a diagnostic agent for the diagnosis of a pre-stage of prostate cancer. The use for production may be *in vivo,* while the use for selection or purification is normally ex *vivo.*

As another alternative aspect, there is provided a use *ex vivo* of an affinity ligand capable of selective interaction with an RBM3 protein as a diagnostic agent for the diagnosis of a pre-stage of prostate cancer.

The various embodiments of aspects one to nine and their benefits apply to the alternative aspect *mutatis mutandis.*

### Examples

### 1. Prostate cancer TMA - prognosis

### a) Material and methods

Tumor material was collected from 88 patients treated with radical prostatectomy for localized prostate cancer at Skåne University Hospital, Malmö, between 1998 and 2003. The median age of patients at the time of surgery was 63 (48-74) years. Written consent was obtained from the patients and the local ethics committee approved the study. Histopathological, clinical and follow-up data were obtained from the clinical- and/or pathology records. Vital status and cause of death was obtained from the Swedish Cause of Death Registry up until December 2006. The mean follow up period was 58.5 months after which three patients had died and 85 patients were alive. Biochemical recurrence (BCR) was defined as PSA > 0.2 ng/ml with a confirmatory level and clinical progression included BCR, death from prostate cancer and adjuvant radiation therapy. During follow up, 14 (15.9%) patients had BCR and 19 (21.6%) patients had clinical progression. Five (5.7%) patients received adjuvant radiotherapy and three (3.4%) patients received adjuvant hormonal therapy.

For tissue microarray (TMA) construction, areas representing cancer and normal prostate tissue were marked on haematoxylin and eosin stained slides. Two 1.00 mm cores from areas with invasive cancer and one 1.00 mm core representing adjacent benign prostate tissue were sampled from each case and mounted in a new recipient block using a manual arraying device (MTA-1 Beecher Instruments Inc., Sun Prairie, WI, USA).

For immunohistochemical analysis, 4 µm TMA-sections were automatically pre-treated using the PT Link system and then stained in an Autostainer Plus (DAKO; Glostrup, Copenhagen, Denmark) with the mouse monoclonal anti-RBM3 antibody 1 B5, obtained as previously described (see Examples, sections 3, 6 and 11 of WO 2010/092190), diluted 1:5000. The RBM3 staining was evaluated by two independent observers who were blinded to clinical and outcome data. The fraction of cells expressing RBM3 in the nucleus (NF) were denoted a score from 0 (0-1%), 1 (2-25%), 2 (26-50%), 3 (51-75%) and 4 (>75%) and the intensity (NI) was scored as 0 (negative/absent), 1 (weak), 2 (moderate) and 3 (strong). A combined nuclear score (NS) of NF x NI, was then constructed, whereby NS = 0-1 was defined as low expression, NS = 2-4 was defined as moderate expression, and NS > 4 was defined as strong expression.

Spearman's rho and Chi-square tests were used for analysis of the correlation between RBM3 expression and relevant clinicopathological characteristics. Kaplan-Meier analysis and log rank test were used to illustrate differences in BCR free time and progression free survival (PFS) according to RBM3 expression. For survival analyses, RBM3 expression (intensity x fraction) was dichotomized into weak vs strong using classification regression tree analysis with BCR as the dependent variable, whereby tumors with a NS ≤2 were denoted as having low RBM3 expression and tumors with a NS >2 as having high RBM3 expression. Cox regression proportional hazards modeling were used to estimate the impact of RBM3 expression on BCR (with RBM3 as a continuous variable) and PFS (with RBM3 as a dichotomized variable) in both uni- and multivariate analysis. Also, survival analysis was performed with RBM3 expression dichotomized using NS = 0 as cut-off, whereby tumors with a NS = 0 were denoted as not expressing RBM3, and tumors with a NS > 0 as expressing RBM3. All tests were two sided. A p-value of 0.05 was considered significant. All statistical analyses were performed using SPSS version 17 (SPSS Inc, Chicago, IL).

### b) Results

While absent or weakly expressed in normal prostatic glands, RBM3 was clearly up-regulated in prostatic intraepithelial neoplasia (PIN), and in invasive carcinoma RBM3 was expressed in various fractions and intensities ranging from negative to strong. There was no obvious heterogeneity in the staining pattern between duplicate tissue cores.

There was no association between RBM3 expression and conventional clinicpathological parameters. Kaplan Meier analysis demonstrated that high expression of RBM3 was associated with a significantly prolonged time to biochemical recurrence (BCR, p = 0.004) and clinical progression (p = 0.004) (Fig 1). These associations were confirmed in Cox univariate analysis (HR 0.56, 95% CI: 0.34-0.93, p = 0.024 for BCR and HR 0.09, 95% CI: 0.01-0.71, p = 0.021 for clinical progression) and remained significant in multivariate analysis, adjusted for preoperative PSA level, Gleason score, presence and or absence of extracapsular extension, seminal vesicle invasion and positive surgical margins (HR 0.41, 95% CI: 0.19-0.89, p = 0.024 for BCR and HR 0.06, 95% CI: 0.01-0.50, p = 0.009 for clinical progression) (the Table). These significant associations were retained in both univariate and multivariate analysis also when the few patients who received adjuvant radiotherapy or hormonal therapy were excluded from the analysis.

**Table. Cox univariate and multivariate analysis of biochemical recurrence (BCR) free survival and progression free survival according to RBM3 expression**

| **RBM3NS dichotomized** | **Progression free survival** | | |
|---|---|---|---|
| | **n (events)** | **HR (95% Cl)** | **p-value** |
| *Univariate* | | | |
| Low (0-2) | 64 (18) | 1.00 | |
| High (3-8) | 24 (1) | 0.09 (0.01-0.71) | 0.021 |
| *Multivariate* | | | |
| Low (0-2) | 64 (16) | 1.00 | |
| High (3-8) | 24 (1) | 0.06 (0.01-0.50) | 0.009 |

| **BCR free survival** | | | |
|---|---|---|---|
| **RBM3 NS continuous** | | **HR (95% CI)** | **p-value** |
| | **n (events)** | | |
| Univariate | 88 (14) | 0.56 (0.34-0.93) | 0.024 |
| Multivariate | 88(12) | 0.41 (0.19-0.89) | 0.024 |

When the patients were split into two groups depending on T-stage (T1 or T2), the association between a high RBM3 expression and prolonged progression free survival remained significant for patients with stage T2 prostate cancer (Figure 2B), and the same trend could be seen for patients with stage T1 prostate cancer (Figure 2A). The association between a high RBM3 expression and prolonged progression free survival as confirmed in Cox univariate analysis (HR: 0.09, 95% CI: 0.01-0.71, p = 0.021) remained significant also after multivariate analysis adjusted for T-stage (HR: 0.085, 95% CI: 0.01-0.64, p = 0.017).

When the alternative cut-off was used, i.e. a NS of 0, the biochemical recurrence free and progression free survival were still significantly better for the RBM3 high group than the RBM3 low group.

### 2. Epitope mapping using Bioplex

### a) Synthetic peptide preparation

A PEPscreen library consisting of 25 biotinylated peptides corresponding to the protein fragment SEQ ID NO:1 of the RBM3 protein (SEQ ID NO:2, SEQ ID NO:3) was synthesized by Sigma-Genosys (Sigma-Aldrich). The peptides were 15 amino acid residues long with a 10 amino acid residues overlap, together covering the entire protein fragment (SEQ ID NO:1). The peptides were resolved in 80 % DMSO to a final concentration of 10 mg/ml. 1.

### b) Bead coupling

Neutravidin (Pierce, Rockford, IL) was immobilized on carboxylated beads (BioPlex COOH Beads, BioRad) in accordance with the manufacturer's protocol. Coupling of 10⁶ beads was performed using a filter membrane bottomed microtiter plate (MultiScreen-HTS, Millipore, Billerica, MA) as previously described (Larsson et al (2009) J Immunol Methods 15;34(1-2):20-32, Schwenk et al (2007) Mol Cell Proteomics 6(1) 125:32). 25 distinct groups of beads with different color code IDs were activated using 1-Ethyl-3-(3-dimethylamino-propyl) carbodiimide and N-Hydroxysuccinimide. Neutravidin (250 µg/ml in 50 mM Hepes pH 7,4) was added to the beads and incubated for 120 min on a shaker. The beads were finally washed, re-suspended, and transferred to micro-centrifuge tubes for storage at 4°C in PBS-BN (1xPBS, 1 %BSA, 0,05% NaN3). All coupled bead populations were treated with sonication in an ultrasonic cleaner (Branson Ultrasonic Corporation, Danbury, CT) for 3 min. The biotinylated peptides were diluted in PBS-BN to a concentration of 0,1 mg/ml, and 50 µl of each peptide was used in the coupling reaction, which was conducted for 60 min with shaking at RT. Finally, the beads were washed with 3 x 100 µl BRE buffer and stored at 4°C until further use.

### c) Determination of binding specificity

A bead mixture containing all 25 bead IDs was prepared and 10 µl of the polyclonal rabbit antibody "anti-RBM3", obtained as described previously (see Examples, section 1 and 2 of WO 2010/092190), was mixed with 30 µl of the bead mix and incubated for 60 min at RT. A filter bottomed microtiter plate (Millipore) was utilized for washing and following each incubation all wells were washed with 2 x 100 µl PBS-BN. To the beads, 25 µl of R-Phycoerythrine labeled anti-rabbit IgG antibody (Jackson ImmunoResearch) was added for a final incubation of 30 min at RT.

Measurements were performed using the Bioplex 200 Suspension Array instrumentation with Bio-Plex Manager 5.0 software. For each experiment, 50 events per bead ID were counted and the median fluorescence intensity (MFI) was used as a measurement of antibody binding to individual bead populations.

### d) Results

The specificities of anti-RBM3 were tested in an assay using beads coupled with synthetic biotinylated peptides. Anti-RBM3 showed strong binding to several of the peptides, among which peptide 6, corresponding to the sequence SEQ ID NO: 20 (a subsequence of SEQ ID NO:1), was one (see figure 4).

### 3. Fractionation of a polyclonal anti-RBM3 antibody

### a) Materials and methods

Peptide specific antibodies were obtained by affinity purification of anti-RMB3 against peptides to which the polyclonal anti-RBM3 antibody was shown to bind in Examples, section 2. Among the peptides chosen was peptide 6, and 600 nmol of biotinylated peptide were diluted with *HiTrap™* Streptavidin binding buffer to a final volume of 1100 µl and applied to 1 ml *HiTrap*™ *Streptavidin* HP columns (GE Healthcare Bio-Sciences AB, Uppsala, Sweden) for binding. After coupling, columns were washed with *HiTrap*™ Streptavidin binding buffer to remove unbound peptides (and a blank run was performed on all columns prior to sample loading.)

Serum obtained from a New Zeeland white rabbit immunized with the recombinant RBM3 fragment SEQ ID NO: 1 fused to a His₆-ABP tag, was purified on a ÄKTAxpress™ (GE Healthcare) liquid chromatography system on eight columns in a serial mode as follows: two 5 ml His₆-ABP columns followed by 5 epitope specific peptide columns and at the end a His₆-ABP-RBM3 fusion protein column. After sample loading, the columns were washed and eluted in parallel to obtain separate antibody fractions. The eluted antibody fractions were epitope mapped using BioPlex, as described above. To further improve the epitope resolution for the antibody fraction that bound peptide 6, alanine scanning of the peptide was performed using the following method: Twenty biotinylated synthetic peptides of the sequence TQRSRGFGFITFTNPEHASV (SEQ ID NO: 21), each with a single alanine mutation introduced at every residue (Sigma-Aldrich) were dissolved in DMSO and diluted to 4 µmoles peptide in 100 µL PBS 7.4 supplemented with 1 mg/mL BSA. The peptides were coupled to 20 Bioplex neutravidin coated beads with 20 unique reporter dyes as described above. The antibody fraction binding to peptide 6 was incubated for one hour in PBST with a cocktail of the different beads consisting of around 15,000 beads per ID. The antibodies were subsequently labeled with PE-conjugated secondary reagent (Moss Inc., USA) and analyzed using Bioplex 200 Suspension Array instrumentation with Bio-Plex Manager 5.0 software.

### b) Results

When the antibody fraction was epitope mapped, the fractionated antibody bound its expected peptide. The antibody fraction that bound to peptide 6 was confirmed to bind the full-length RBM3 protein (SEQ ID NO:2) by IHC and Western Blot analysis. Preserved antibody binding for the fraction that bound peptide 6 was observed for all amino acid positions except the alanine-substitutions Phe12Ala, Thr13Ala, and Asn14Ala of the epitope. The epitope for the antibody fraction was thus determined to include the sequence FTN (SEQ ID NO:22) within SEQ ID NO: 4 (see figure 4).

### 4. Generation of monoclonal antibodies.

### a) Materials and methods

A synthetic peptide (SEQ ID NO: 23), including peptide 6 (SEQ ID NO: 20) and peptide 7 (SEQ ID NO:24) in Section 2 and having a cystein residue added at its N-terminal to which BSA was coupled according to standard procedure, was used as antigen for production of monoclonal antibodies. The antigen was injected subcutaneously into BALB/c mice (4-6 weeks old, female) at three week intervals. The antigen was mixed with complete Freund's adjuvant for the first injection and incomplete Freund's adjuvant for the following injections. Three days before fusion, the mouse was last challenged with antigen intravenously. Hybridomas were generated by fusion of mouse splenocytes with the Sp2/0 myeloma cell line. By screening several cell lines using ELISA, cells that secreted antibodies specific for the antigen (SEQ ID N0:1) were identified. Cell lines that showed positive results in ELISA, Western blot (WB) and immunohistochemistry (IHC) were selected for subcloning.

In addition, the immunohistochemical staining patterns of the monoclonal antibodies were compared to that of anti-RBM3.

### b) Results

Cell-lines were screened by ELISA to identify lines that produce monoclonal antibodies (mAbs) that recognize the antigen (SEQ ID N0:1), but not the fusion tag, BSA. There were 37 cell-lines showing specific binding to the antigen SEQ ID NO:1 in ELISA and these were selected for further testing. For each of the selected 37 clones 150 - 300 µl supernatant was collected and azide was added. The supernatants were stored at +4°C. Further testing of the cell lines showed that clones denoted 7F5, 10F1, 12A10, 12C9, and 14D9 gave positive results in both Western blot and IHC analysis. These clones were selected for subcloning and expansion.

### 5. Evaluation of antibody specificity

### a) Material and methods

The specificity of the polyclonal antibody (anti-RBM3), obtained as previously described, and the monoclonal antibodies (7F5, 10F1, 12A10, 12C9, and 14D9), obtained as described in Section 4 above, were analyzed by Western Blot. Western blot was performed by separation of total protein extracts from the human cell line RT4 on 4-20 % criterion TGX prep well gels under reducing conditions, followed by electro-transfer to PVDF membranes (Millipore) according to the manufacturer's recommendations. The membranes were blocked (5 % milk in 1x TBST (0.1% Tween20)) for 1 h at room temperature, incubated with the primary monoclonal antibody (diluted 1:10 in 1 % BSA, 1xTBST) and washed in PBST. The secondary HRP-conjugated antibody (polyclonal goat anti-mouse or polyclonal swine anti-rabbit, both Dako) was diluted 1:3000 in blocking buffer and chemiluminescence detection was carried out using a CCD camera (Syngene) and Immobilon Western Chemiluminescent HRP Substrate (Millipore), according to the manufacturer's protocol.

### b) Results

The results of the Western blot analysis shows that the antibodies specifically detect a band of approximately 16 kDa in the RT4 cell line. The theoretical molecular weight of RBM3 is 16 kDa (as calculated from the RBM3 amino acid sequence SEQ ID NO:2), corresponding well to the result obtained. Additional bands were observed for anti-RBM3. Overall, the results show that the monoclonal antibodies were more specific than the polyclonal antibody, (see figure 5).

### 6. Epitope mapping of monoclonal antibodies

### a) Material and methods

The monoclonal antibodies obtained as described in Section 5, were epitope mapped using Bioplex. Synthetic peptide preparation and bead coupling was performed as described in Section 2. A bead mixture containing all 25 bead IDs was prepared and 10 µl of the monoclonal antibodies, diluted 1:10 in PBS-BN (1 % BSA), was mixed with 5 µl of the bead mix and incubated for 60 min at RT. A filter bottomed microtiter plate (Millipore) was utilized for washing and following each incubation all wells were washed with 3 x 100 µl PBST. 25 µl of PE-conjugated goat anti-mouse IgG (Jackson ImmunoResearch) were added (4 ug/ml) for a final incubation of 60 min at RT.

Measurements were performed using BioPlex 200 Suspension Array instrumentation with Bio-Plex Manager 5.0 software. For each experiment 50 events per bead ID were counted and the median fluorescence intensity (MFI) was used as a measurement of antibody binding to individual bead populations.

### b) Results

The specificities of the monoclonal antibodies were tested in an assay using beads coupled with synthetic biotinylated peptides. All monoclonals tested showed strong binding to two of the peptides, namely peptide 7 (SEQ ID NO: 24) and 8, SEQ ID NO: 25 (see figure 4) corresponding to the consensus sequence SEQ ID NO: 26 within SEQ ID NO:4.

### 7. A non-limiting example of an establishment of a prognosis

A prostate cancer diagnosis for a patient is established based on the morphological examination of tissue material from prostate biopsies. This tissue material is also used to provide one or more sample(s) for the prognostic method. Further, for the provision of a "negative reference", a sample is taken from archival material comprising tissue essentially lacking RBM3 protein expression. Such archival tissue may for example be prostate cancer tissue having a pre-established absent RBM3 protein expression. Further, for the provision of a "positive reference", a sample is taken from archival material comprising tissue having high RBM3 protein expression, such as prostate cancer tissue having a pre-established high RBM3 protein expression level.

The sample material is fixated in buffered formalin and histo-processed in order to obtain thin sections (e.g. 4 µm) of the sample material.

Immunohistochemistry is performed in accordance with the Examples of WO 2010/092190. One or more sample sections from each sample is/are mounted on glass slides that are incubated for 45 min in 60 °C, deparaffinized (if the sample in question was paraffinized) in xylene (2 x 15 min) and hydrated in graded alcohols. For antigen retrieval, slides are immersed in TRS (Target Retrieval Solution, pH 6.0, DakoCytomation) and boiled for 4 min at 125 °C in a Decloaking chamber® (Biocare Medical). Slides are placed in the Autostainer® (DakoCytomation) and endogenous peroxidase is initially blocked with H₂O₂ (DakoCytomation). The reason for mounting multiple sample sections is to increase the accuracy of the results.

The monoclonal antibody 1 B5 (see above) is added to the slides, which are incubated for 30 min in room temperature, followed by 30 min of incubation in room temperature with a labeled secondary antibody; e.g. goat-anti-peroxidase (rabbit or mouse) conjugated Envision®. To detect the secondary antibody, diaminobenzidine (DakoCytomation) is used as chromogen, contrasted with a Harris hematoxylin (Sigma-Aldrich) counterstaining. Between all steps, slides are rinsed in wash buffer (DakoCytomation). The slides are then mounted with Pertex® (Histolab) mounting media.

As a tool to validate the staining procedure, two control cell-lines may be used; e.g. one slide with cells expressing RBM3 protein (positive cell line) and one slide having cells with indistinct weak or no RBM3 protein expression (negative cell line). The skilled artisan understands how to provide such cell lines, for example guided by the disclosure of Rhodes et al. (2006) The biomedical scientist, p 515-520. The control-line slides may be simultaneously stained in the same procedure as the other slides, i.e. incubated with the same primary and secondary antibodies.

For example, the tumor tissue slides from the subject, the staining reference slides, and optionally, the slides with control cell-lines, may be scanned in a light microscope using a ScanScope T2 automated slide scanning system (Aperio Technologies) at x20 magnification. However, this scanning step is not necessary, but may make the procedure easier if, for example, the preparation and staining of the slides and the evaluation of the stained slides (see below) are performed at different locations or by different persons.

If control cell-lines are used, these are inspected to validate the staining procedure. If the cell-lines display staining results outside acceptable criteria, e.g. staining artifacts recognized by the skilled artisan, the staining of the tissue samples is considered invalid and the whole staining procedure is repeated with new slides. If the positive and negative cell-lines display strong staining intensity and indistinct weak or no staining intensity, respectively, the staining is considered as valid.

The stained sample slide(s) from the tumor tissue sample from the patient is/are manually evaluated by visual inspection, and a sample value (NS) of each slide is determined according to the above. The person performing the evaluation and determination is aided by visual inspection of the stained positive and negative reference slides.

The sample value(s) from the tumor tissue sample from the patient is/are then compared to a reference value. If more than one sample slide are evaluated and thereby more than one sample value are obtained, the sample value that is compared to the reference value may be a mean or median value of the obtained sample values.

The reference value may be a NS of 2. In such case it is concluded that the tested patient belongs to a group of patients having a relatively good prognosis if the NS of the sample(s) is higher than 2 and a group of patients having a relatively poor prognosis if the NS of the sample(s) is 2 or lower. The prognoses of the respective groups, which preferably consists of patients having prostate cancers of the same stage as the prostate cancer of the patient of the method, may be read from dichotomized data as those presented in the figures, wherein the upper curve represents the group of patients having the relatively good prognosis and the lower curve represents the group of patients having the relatively poor prognosis. For example, the relatively good stage-independent prognosis may be an average five-year progression free survival of about 86 % and the relatively poor stage-independent prognosis may be an average five-year biochemical survival of about 50 % (figure 2B).

All cited material, including but not limited to publications, DNA or protein data entries, and patents, referred to in this application are herein incorporated by reference.

The invention being thus described, it will be obvious that the same may be varied in many ways. Such variations are not to be regarded as a departure from the spirit and scope of the present invention, and all such modifications as would be obvious to one skilled in the art are intended to be included within the scope of the following claims.

## Claims

1. Method of determining whether a mammalian subject having a prostate cancer belongs to a first or a second group, wherein the prognosis of subjects of the first group is better than the prognosis of subjects of the second group, comprising the steps of:
a) evaluating an amount of RBM3 in at least part of an earlier obtained sample comprising tumor cells from the prostate of said subject and determining a sample value corresponding to the evaluated amount;
b) comparing said sample value with a predetermined reference value;
and
if said sample value is higher than said reference value,
c1) concluding that the subject belongs to the first group; and
if said sample value is lower than or equal to said reference value,
c2) concluding that the subject belongs to the second group.

2. Method according to claim 1, wherein the prognosis is a stage-independent prognosis.

3. Non-treatment strategy method for a mammalian subject having a prostate cancer, comprising the steps of:
a) evaluating an amount of RBM3 in at least part of an earlier obtained sample comprising tumor cells from the prostate of said subject and determining a sample value corresponding to the evaluated amount;
b) comparing the sample value with a predetermined reference value; and if the sample value is higher than the reference value,
c) refraining from treating the subject with a prostate cancer treatment regimen.

4. Method of determining whether a mammalian subject is not in need of a prostate cancer treatment regimen, comprising the steps of:
a) evaluating an amount of RBM3 in at least part of an earlier obtained sample comprising tumor cells from the prostate of said subject and determining a sample value corresponding to the evaluated amount;
b) comparing the sample value with a predetermined reference value; and if the sample value is higher than the reference value,
c) concluding that the subject is not in need of said prostate cancer treatment regimen.

5. Method according to any one of the preceding claims, wherein the first and the second group consists of subjects having prostate cancer of a stage which is the same as the stage of the prostate cancer of the subject of the method.

6. Method according to any one of the preceding claims, wherein the subject has a PSA concentration of at least 10 ng/ml and/or a Gleason sum of 5-8.

7. Method according to any one of the preceding claims, wherein the TNM stage of the prostate cancer is T2-4, N0, M0.

8. Method according to any one of the preceding claims, wherein said sample is obtained from a biopsy or surgically removed tissue.

9. Method according to any one of claims 2-8, wherein said prostate cancer treatment regimen is selected from the group consisting of prostatectomy, cryosurgery, high-intensity focused ultrasound treatment and radiation therapy.

10. Method according to any one of claims 2-8, wherein said prostate cancer treatment regimen is an adjuvant treatment.

11. Method according to claim 10, wherein said adjuvant treatment is selected from the group consisting of chemotherapy, hormone therapy and adjuvant radiation therapy.

12. Use ex *vivo* of an RBM3 protein as a prognostic marker for prostate cancer.

13. Use *ex vivo* of an affinity ligand capable of selective interaction with an RBM3 protein as a diagnostic agent for the diagnosis of a pre-stage of prostate cancer.

14. RBM3 protein fragment, which consists of 17 amino acid residues or less and comprises a sequence selected from SEQ ID NO: 22, 24, 25 and 26.

15. Affinity ligand capable of selective interaction with an RBM3 protein fragment consisting of 21 amino acid residues or less and comprising a sequence selected from SEQ ID NO: 22-26.
